# EUROPEAN PATENT SPECIFICATION

(11) **EP 1 648 996 B1**
(45) Date of publication and mention of the grant of the patent: **14.03.2012**
(21) Application number: 04756144.4
(22) Date of filing: 25.06.2004
(51) Int. Cl.: C12N 9/26, C12N 15/56

(54) **ENZYMES FOR STARCH PROCESSING**
ENZYME ZUR STÄRKEVERARBEITUNG
ENZYMES DE TRAITEMENT D'AMIDON

(30) Priority: 25.06.2003 DK 200300949; 25.06.2003 US 482589 P; 29.07.2003 US 490751 P; 14.10.2003 US 511044 P; 24.10.2003 DK 200301568; 27.10.2003 US 514854 P; 10.05.2004 US 569862 P
(43) Date of publication of application: 26.04.2006
(60) Divisional application: 10181122.2
(73) Proprietor: Novozymes A/S, 2880 Bagsvaerd (DK); Novozymes North America, Inc., Franklinton, NC 27525 (US)
(72) Inventor: TAIRA, Rikako, Tokyo 130-0012 (JP); TKAGI, Shinobu, Funabashi, Chiba 274-0825 (JP); HJORT, Carsten, DK-3500 Vaerloese (DK); VIKSO-NIELSEN, Anders, DK-3550 Slangerup (DK); ALLAIN, Eric, Boone, NC 28607-8941 (US); UDAGAWA, Hiroaki, Nakohama-shi, Yokohama-shi, 231-0045 (JP); FUKUYAMA, Shiro, Narashino-shi, Chiba 275-0017 (JP); MATSUI, Tomoko, Ichikawa, Chiba 272-0026 (JP)
(74) Representative: Kofoed, Gertrud Sonne
(86) International application number: PCT/US2004/020499
(87) International publication number: WO 2005/003311

### SEQUENCE LISTING

<110> Udagawa, Hiroaki
   Taira, Rikako
   Tkagi, Shinobu
   Allain, Eric
   Hjort, Carsten
   Vikso-Nielsen, Anders
<120> HYBRID ENZYMES FOR STARCH PROCESSING
<130> 10490.500-US
<160> 43
<170> PatentIn version 3.2
<210> 1
   <211> 40
   <212> DNA
   <213> Artificial
<220>
   <223> Primer
<400> 1
   gaagggatcc gatttttact agtacatcca aagccaccac 40
<210> 2
   <211> 37
   <212> DNA
   <213> Artificial
<220>
   <223> Primer
<400> 2
   tttgtcgacc tacctccacg tatcaaccac cgtctcc 37
<210> 3
   <211> 39
   <212> DNA
   <213> Artificial
<220>
   <223> Primer
<400> 3
   tttggatcca ccatgagatt atcgacttcg agtctcttc 39
<210> 4
   <211> 38
   <212> DNA
   <213> Artificial
<220>
   <223> Primer
<400> 4 tttactagta gcagcagcag ttgtggtcgt ggttgttc 38
<210> 5
   <211> 396
   <212> DNA
   <213> Aspergillus kawachii
<220>
   <221> CDS
   <222> (1)..(396)
<400> 5
   act agt aca tcc aaa gcc acc acc tcc tct tct tct tct tct gct gct        48
   Thr Ser Thr Ser Lys Ala Thr Thr Ser Ser Ser Ser Ser Ser Ala Ala
   1               5                   10                  15
   gct act act tct tca tca tgc acc gca aca agc acc acc ctc ccc atc        96
   Ala Thr Thr Ser Ser Ser Cys Thr Ala Thr Ser Thr Thr Leu Pro Ile
               20                  25                  30
   acc ttc gaa gaa ctc gtc acc act acc tac ggg gaa gaa gtc tac ctc       144
   Thr Phe Glu Glu Leu Val Thr Thr Thr Tyr Gly Glu Glu Val Tyr Leu
           35                  40                  45
   agc gga tct atc tcc cag ctc gga gag tgg gat acg agt gac gcg gtg       192
   Ser Gly Ser Ile Ser Gln Leu Gly Glu Trp Asp Thr Ser Asp Ala Val
       50                  55                  60
   aag ttg tcc gcg gat gat tat acc tcg agt aac ccc gag tgg tct gtt       240
   Lys Leu Ser Ala Asp Asp Tyr Thr Ser Ser Asn Pro Glu Trp Ser Val
   65                  70                  75                  80
   act gtg tcg ttg ccg gtg ggg acg acc ttc gag tat aag ttt att aag       288
   Thr Val Ser Leu Pro Val Gly Thr Thr Phe Glu Tyr Lys Phe Ile Lys
                   85                  90                  95
   gtc gat gag ggt gga agt gtg act tgg gaa agt gat ccg aat agg gag       336
   Val Asp Glu Gly Gly Ser Val Thr Trp Glu Ser Asp Pro Asn Arg Glu
               100                 105                 110
   tat act gtg cct gaa tgt ggg aat ggg agt ggg gag acg gtg gtt gat       384
   Tyr Thr Val Pro Glu Cys Gly Asn Gly Ser Gly Glu Thr Val Val Asp
           115                 120                 125
   acg tgg agg tag                                                       396
   Thr Trp Arg
       130
<210> 6
   <211> 131
   <212> PRT
   <213> Aspergillus kawachii
<400> 6 <210> 7
   <211> 1533
   <212> DNA
   <213> Aspergillus niger
<220>
   <221> CDS
   <222> (1)..(1533)
<400> 7
<210> 8
   <211> 511
   <212> PRT
   <213> Aspergillus niger
<400> 8
   Met Arg Leu Ser Thr Ser Ser Leu Phe Leu Ser Val Ser Leu Leu Gly
   1               5                   10                  15
   Lys Leu Ala Leu Gly Leu Ser Ala Ala Glu Trp Arg Thr Gln Ser Ile
               20                  25                  30
   Tyr Phe Leu Leu Thr Asp Arg Phe Gly Arg Thr Asp Asn Ser Thr Thr
           35                  40                  45
   Ala Thr Cys Asp Thr Gly Asp Gln Ile Tyr Cys Gly Gly Ser Trp Gln
       50                  55                  60
   Gly Ile Ile Asn His Leu Asp Tyr Ile Gln Gly Met Gly Phe Thr Ala
   65                  70                  75                  80
   Ile Trp Ile Ser Pro Ile Thr Glu Gln Leu Pro Gln Asp Thr Ala Asp
                   85                  90                  95
   Gly Glu Ala Tyr His Gly Tyr Trp Gln Gln Lys Ile Tyr Asp Val Asn
               100                 105                 110
   Ser Asn Phe Gly Thr Ala Asp Asp Leu Lys Ser Leu Ser Asp Ala Leu
           115                 120                 125
   His Ala Arg Gly Met Tyr Leu Met Val Asp Val Val Pro Asn His Met
       130                 135                 140
   Gly Tyr Ala Gly Asn Gly Asn Asp Val Asp Tyr Ser Val Phe Asp Pro
   145                 150                 155                 160
   Phe Asp Ser Ser Ser Tyr Phe His Pro Tyr Cys Leu Ile Thr Asp Trp
                   165                 170                 175
   Asp Asn Leu Thr Met Val Gln Asp Cys Trp Glu Gly Asp Thr Ile Val
               180                 185                 190
   Ser Leu Pro Asp Leu Asn Thr Thr Glu Thr Ala Val Arg Thr Ile Trp
           195                 200                 205
   Tyr Asp Trp Val Ala Asp Leu Val Ser Asn Tyr Ser Val Asp Gly Leu
       210                 215                 220
   Arg Ile Asp Ser Val Leu Glu Val Glu Pro Asp Phe Phe Pro Gly Tyr
   225                 230                 235                 240
   Gln Glu Ala Ala Gly Val Tyr Cys Val Gly Glu Val Asp Asn Gly Asn
                   245                 250                 255
   Pro Ala Leu Asp Cys Pro Tyr Gln Lys Val Leu Asp Gly Val Leu Asn
               260                 265                 270
   Tyr Pro Ile Tyr Trp Gln Leu Leu Tyr Ala Phe Glu Ser Ser Ser Gly
           275                 280                 285
   Ser Ile Ser Asn Leu Tyr Asn Met Ile Lys Ser Val Ala Ser Asp Cys
       290                 295                 300
   Ser Asp Pro Thr Leu Leu Gly Asn Phe Ile Glu Asn His Asp Asn Pro
   305                 310                 315                 320
   Arg Phe Ala Ser Tyr Thr Ser Asp Tyr Ser Gln Ala Lys Asn Val Leu
                   325                 330                 335
   Ser Tyr Ile Phe Leu Ser Asp Gly Ile Pro Ile Val Tyr Ala Gly Glu
               340                 345                 350
   Glu Gln His Tyr Ser Gly Gly Lys Val Pro Tyr Asn Arg Glu Ala Thr
           355                 360                 365
   Trp Leu Ser Gly Tyr Asp Thr Ser Ala Glu Leu Tyr Thr Trp Ile Ala
       370                 375                 380
   Thr Thr Asn Ala Ile Arg Lys Leu Ala Ile Ser Ala Asp Ser Ala Tyr
   385                 390                 395                 400
   Ile Thr Tyr Ala Asn Asp Ala Phe Tyr Thr Asp Ser Asn Thr Ile Ala
                   405                 410                 415
   Met Arg Lys Gly Thr Ser Gly Ser Gln Val Ile Thr Val Leu Ser Asn
               420                 425                 430
   Lys Gly Ser Ser Gly Ser Ser Tyr Thr Leu Thr Leu Ser Gly Ser Gly
           435                 440                 445
   Tyr Thr Ser Gly Thr Lys Leu Ile Glu Ala Tyr Thr Cys Thr Ser Val
       450                 455                 460
   Thr Val Asp Ser Ser Gly Asp Ile Pro Val Pro Met Ala Ser Gly Leu
   465                 470                 475                 480
   Pro Arg Val Leu Leu Pro Ala Ser Val Val Asp Ser Ser Ser Leu Cys
                   485                 490                 495
   Gly Gly Ser Gly Arg Thr Thr Thr Thr Thr Thr Ala Ala Thr Ser
   500 505 510
<210> 9
   <211> 102
   <212> PRT
   <213> Bacillus flavothermus
<400> 9
   Ile Ser Thr Thr Ser Gln Ile Thr Phe Thr Val Asn Asn Ala Thr Thr
   1 5 10 15
   Val Trp Gly Gln Asn Val Tyr Val Val Gly Asn Ile Ser Gln Leu Gly
   20 25 30
   Asn Trp Asp Pro Val His Ala Val Gln Met Thr Pro Ser Ser Tyr Pro
   35 40 45
   Thr Trp Thr Val Thr Ile Pro Leu Leu Gln Gly Gln Asn Ile Gln Phe
   50 55 60
   Lys Phe Ile Lys Lys Asp Ser Ala Gly Asn Val Ile Trp Glu Asp Ile
   65 70 75 80
   Ser Asn Arg Thr Tyr Thr Val Pro Thr Ala Ala Ser Gly Ala Tyr Thr
   85 90 95
   Ala Ser Trp Asn Val Pro
   100
<210> 10
   <211> 99
   <212> PRT
   <213> Bacillus sp.
<400> 10
   Thr Ser Asn Val Thr Phe Thr Val Asn Asn Ala Thr Thr Val Tyr Gly
   1 5 10 15
   Gln Asn Val Tyr Val Val Gly Asn Ile Pro Glu Leu Gly Asn Trp Asn
   20 25 30
   Ile Ala Asn Ala Ile Gln Met Thr Pro Ser Ser Tyr Pro Thr Trp Lys
   35 40 45
   Thr Thr Val Ser Leu Pro Gln Gly Lys Ala Ile Glu Phe Lys Phe Ile
   50 55 60
   Lys Lys Asp Ser Ala Gly Asn Val Ile Trp Glu Asn Ile Ala Asn Arg
   65 70 75 80
   Thr Tyr Thr Val Pro Phe Ser Ser Thr Gly Ser Tyr Thr Ala Asn Trp
   85 90 95
   Asn Val Pro
<210> 11
   <211> 102
   <212> PRT
   <213> Alcaliphilic Bacillus
<400> 11
   Thr Ser Thr Thr Ser Gln Ile Thr Phe Thr Val Asn Asn Ala Thr Thr
   1 5 10 15
   Val Trp Gly Gln Asn Val Tyr Val Val Gly Asn Ile Ser Gln Leu Gly
   20 25 30
   Asn Trp Asp Pro Val Asn Ala Val Gln Met Thr Pro Ser Ser Tyr Pro
   35 40 45
   Thr Trp Val Val Thr Val Pro Leu Pro Gln Ser Gln Asn Ile Gln Phe
   50 55 60
   Lys Phe Ile Lys Lys Asp Gly Ser Gly Asn Val Ile Trp Glu Asn Ile
   65 70 75 80
   Ser Asn Arg Thr Tyr Thr Val Pro Thr Ala Ala Ser Gly Ala Tyr Thr
   85 90 95
   Ala Asn Trp Asn Val Pro
   100
<210> 12
   <211> 112
   <212> PRT
   <213> Hormoconis resinae
<400> 12
   Cys Gln Val Ser Ile Thr Phe Asn Ile Asn Ala Thr Thr Tyr Tyr Gly
   1 5 10 15
   Glu Asn Leu Tyr Val Ile Gly Asn Ser Ser Asp Leu Gly Ala Trp Asn
   20 25 30
   Ile Ala Asp Ala Tyr Pro Leu Ser Ala Ser Ala Tyr Thr Gln Asp Arg
           35                  40                  45
   Pro Leu Trp Ser Ala Ala Ile Pro Leu Asn Ala Gly Glu Val Ile Ser
       50                  55                  60
   Tyr Gln Tyr Val Arg Gln Glu Asp Cys Asp Gln Pro Tyr Ile Tyr Glu
   65                  70                  75                  80
   Thr Val Asn Arg Thr Leu Thr Val Pro Ala Cys Gly Gly Ala Ala Val
                   85                  90                  95
   Thr Thr Asp Asp Ala Trp Met Gly Pro Val Gly Ser Ser Gly Asn Cys
               100                 105                 110
<210> 13
   <211> 95
   <212> PRT
   <213> Lentinula edodes
<400> 13
   Val Ser Val Thr Phe Asn Val Asp Ala Ser Thr Leu Glu Gly Gln Asn
   1               5                   10                  15
   Val Tyr Leu Thr Gly Ala Val Asp Ala Leu Glu Asp Trp Ser Thr Asp
               20                  25                  30
   Asn Ala Ile Leu Leu Ser Ser Ala Asn Tyr Pro Thr Trp Ser Val Thr
           35                  40                  45
   Val Asp Leu Pro Gly Ser Thr Asp Val Gln Tyr Lys Tyr Ile Lys Lys
       50                  55                  60
   Asp Gly Ser Gly Thr Val Thr Trp Glu Ser Asp Pro Asn Met Glu Ile
   65                  70                  75                  80
   Thr Thr Pro Ala Asn Gly Thr Tyr Ala Thr Asn Asp Thr Trp Arg
                   85                  90                  95
<210> 14
   <211> 107
   <212> PRT
   <213> Neurospora crassa
<400> 14
   Cys Ala Ala Asp His Glu Val Leu Val Thr Phe Asn Glu Lys Val Thr
   1               5                   10                  15
   Thr Ser Tyr Gly Gln Thr Val Lys Val Val Gly Ser Ile Ala Ala Leu
               20                  25                  30
   Gly Asn Trp Ala Pro Ala Ser Gly Val Thr Leu Ser Ala Lys Gln Tyr
           35                  40                  45
   Ser Ser Ser Asn Pro Leu Trp Ser Thr Thr Ile Ala Leu Pro Gln Gly
       50                  55                  60
   Thr Ser Phe Lys Tyr Lys Tyr Val Val Val Asn Ser Asp Gly Ser Val
   65                  70                  75                  80
   Lys Trp Glu Asn Asp Pro Asp Arg Ser Tyr Ala Val Gly Thr Asp Cys
                   85                  90                  95
   Ala Ser Thr Ala Thr Leu Asp Asp Thr Trp Arg
               100                 105
<210> 15
   <211> 115
   <212> PRT
   <213> Talaromyces byssochlamydioides
<400> 15
   Thr Thr Thr Gly Ala Ala Pro Cys Thr Thr Pro Thr Thr Val Ala Val
   1               5                   10                  15
   Thr Phe Asp Glu Ile Val Thr Thr Thr Tyr Gly Glu Thr Val Tyr Leu
               20                  25                  30
   Ser Gly Ser Ile Pro Ala Leu Gly Asn Trp Asp Thr Ser Ser Ala Ile
           35                  40                  45
   Ala Leu Ser Ala Val Asp Tyr Thr Ser Ser Asn Pro Leu Trp Tyr Val
       50                  55                  60
   Thr Val Asn Leu Pro Ala Gly Thr Ser Phe Glu Tyr Lys Phe Phe Val
   65                  70                  75                  80
   Gln Gln Thr Asp Gly Thr Ile Val Trp Glu Asp Asp Pro Asn Arg Ser
                   85                  90                  95
   Tyr Thr Val Pro Ala Asn Cys Gly Gln Thr Thr Ala Ile Ile Asp Asp
               100                 105                 110
   Ser Trp Gln
           115
<210> 16
   <211> 115
   <212> PRT
   <213> Geosmithia cylindrospora:
<400> 16
   Thr Ser Thr Gly Ser Ala Pro Cys Thr Thr Pro Thr Thr Val Ala Val
   1               5                   10                  15
   Thr Phe Asp Glu Ile Val Thr Thr Ser Tyr Gly Glu Thr Val Tyr Leu
               20                  25                  30
   Ala Gly Ser Ile Ala Ala Leu Gly Asn Trp Asp Thr Asn Ser Ala Ile
           35                  40                  45
   Ala Leu Ser Ala Ala Asp Tyr Thr Ser Asn Asn Asn Leu Trp Tyr Val
       50                  55                  60
   Thr Val Asn Leu Ala Ala Gly Thr Ser Phe Gln Tyr Lys Phe Phe Val
   65                  70                  75                  80
   Lys Glu Thr Asp Ser Thr Ile Val Trp Glu Asp Asp Pro Asn Arg Ser
                   85                  90                  95
   Tyr Thr Val Pro Ala Asn Cys Gly Gln Thr Thr Ala Ile Ile Asp Asp
               100                 105                 110
   Thr Trp Gln
           115
<210> 17
   <211> 139
   <212> PRT
   <213> Scorias spongiosa
<400> 17
   Ala Lys Val Pro Ser Thr Cys Ser Ala Ser Ser Ala Thr Gly Thr Cys
   1               5                   10                  15
   Thr Thr Ala Thr Ser Thr Phe Gly Gly Ser Thr Pro Thr Thr Ser Cys
               20                  25                  30
   Ala Thr Thr Pro Thr Leu Thr Thr Val Leu Phe Asn Glu Arg Ala Thr
           35                  40                  45
   Thr Asn Phe Gly Gln Asn Val His Leu Thr Gly Ser Ile Ser Gln Leu
       50                  55                  60
   Gly Ser Trp Asp Thr Asp Ser Ala Val Ala Leu Ser Ala Val Asn Tyr
   65                  70                  75                  80
   Thr Ser Ser Asp Pro Leu Trp Phe Val Arg Val Gln Leu Pro Ala Gly
                   85                  90                  95
   Thr Ser Phe Gln Tyr Lys Tyr Phe Lys Lys Asp Ser Ser Asn Ala Val
               100                 105                 110
   Ala Trp Glu Ser Asp Pro Asn Arg Ser Tyr Thr Val Pro Leu Asn Cys
           115                 120                 125
   Ala Gly Thr Ala Thr Glu Asn Asp Thr Trp Arg
       130                 135
<210> 18
   <211> 126
   <212> PRT
   <213> Eupenicillium ludwigii
<400> 18
   Ser Thr Thr Thr Thr Ser Thr Thr Lys Thr Thr Thr Thr Ser Thr Thr
   1               5                   10                  15
   Thr Ser Cys Thr Thr Pro Thr Ala Val Ala Val Thr Phe Asp Leu Ile
               20                  25                  30
   Ala Thr Thr Tyr Tyr Gly Glu Asn Ile Lys Ile Ala Gly Ser Ile Ser
           35                  40                  45
   Gln Leu Gly Asp Trp Asp Thr Ser Asn Ala Val Ala Leu Ser Ala Ala
       50                  55                  60
   Asp Tyr Thr Ser Ser Asp His Leu Trp Phe Val Asp Ile Asp Leu Pro
   65                  70                  75                  80
   Ala Gly Thr Val Phe Glu Tyr Lys Tyr Ile Arg Ile Glu Ser Asp Gly
                   85                  90                  95
   Ser Ile Glu Trp Glu Ser Asp Pro Asn Arg Ser Tyr Thr Val Pro Ala
               100                 105                 110
   Ala Cys Ala Thr Thr Ala Val Thr Glu Asn Asp Thr Trp Arg
   115 120 125
<210> 19
   <211> 116
   <212> PRT
   <213> Aspergillus japonicus
<400> 19
   Lys Thr Ser Thr Thr Thr Ser Ser Cys Ser Thr Pro Thr Ser Val Ala
   1 5 10 15
   Val Thr Phe Asp Val Ile Ala Thr Thr Thr Tyr Gly Glu Asn Val Tyr
   20 25 30
   Ile Ser Gly Ser Ile Ser Gln Leu Gly Ser Trp Asp Thr Ser Ser Ala
   35 40 45
   Ile Ala Leu Ser Ala Ser Gln Tyr Thr Ser Ser Asn Asn Leu Trp Tyr
   50 55 60
   Ala Thr Val His Leu Pro Ala Gly Thr Thr Phe Gln Tyr Lys Tyr Ile
   65 70 75 80
   Arg Lys Glu Thr Asp Gly Ser Val Thr Trp Glu Ser Asp Pro Asn Arg
   85 90 95
   Ser Tyr Thr Val Pro Ser Ser Cys Gly Val Ser Ser Ala Thr Glu Ser
   100 105 110
   Asp Thr Trp Arg
   115
<210> 20
   <211> 133
   <212> PRT
   <213> Penicillium cf. miczynskii
<400> 20
   Thr Thr Thr Gly Gly Thr Thr Thr Ser Gln Gly Ser Thr Thr Thr Thr
   1 5 10 15
   Ser Lys Thr Ser Thr Thr Thr Ser Ser Cys Thr Ala Pro Thr Ser Val
   20 25 30
   Ala Val Thr Phe Asp Leu Ile Ala Thr Thr Val Tyr Asp Glu Asn Val
   35 40 45
   Gln Leu Ala Gly Ser Ile Ser Ala Leu Gly Ser Trp Asp Thr Ser Ser
       50                  55                  60
   Ala Ile Arg Leu Ser Ala Ser Gln Tyr Thr Ser Ser Asn His Leu Trp
   65                  70                  75                  80
   Tyr Val Ala Val Ser Leu Pro Ala Gly Gln Val Phe Gln Tyr Lys Tyr
                   85                  90                  95
   Ile Arg Val Ala Ser Ser Gly Thr Ile Thr Trp Glu Ser Asp Pro Asn
               100                 105                 110
   Leu Ser Tyr Thr Val Pro Val Ala Cys Ala Ala Thr Ala Val Thr Ile
           115                 120                 125
   Ser Asp Thr Trp Arg
       130
<210> 21
   <211> 116
   <212> PRT
   <213> Mz1 Penicillium sp.
<400> 21
   Thr Lys Thr Ser Thr Ser Thr Ser Cys Thr Thr Pro Thr Ala Val Ala
   1               5                   10                  15
   Val Thr Phe Asp Leu Ile Ala Thr Thr Thr Tyr Gly Glu Asn Ile Lys
               20                  25                  30
   Ile Ala Gly Ser Ile Ala Ala Leu Gly Ala Trp Asp Thr Asp Asp Ala
           35                  40                  45
   Val Ala Leu Ser Ala Ala Asp Tyr Thr Asp Ser Asp His Leu Trp Phe
       50                  55                  60
   Val Thr Gln Ser Ile Pro Ala Gly Thr Val Phe Glu Tyr Lys Tyr Ile
   65                  70                  75                  80
   Arg Val Glu Ser Asp Gly Thr Ile Glu Trp Glu Ser Asp Pro Asn Arg
                   85                  90                  95
   Ser Tyr Thr Val Pro Ala Ala Cys Ala Thr Thr Ala Val Thr Glu Ser
               100                 105                 110
   Asp Thr Trp Arg
   115
<210> 22
   <211> 114
   <212> PRT
   <213> Thysanophora sp
<400> 22
   Phe Thr Ser Thr Thr Lys Thr Ser Cys Thr Thr Pro Thr Ser Val Ala
   1               5                   10                  15
   Val Thr Phe Asp Leu Ile Ala Thr Thr Thr Tyr Gly Glu Ser Ile Arg
               20                  25                  30
   Leu Val Gly Ser Ile Ser Glu Leu Gly Asp Trp Asp Thr Gly Ser Ala
           35                  40                  45
   Ile Ala Leu His Ala Thr Asp Tyr Thr Asp Ser Asp His Leu Trp Phe
       50                  55                  60
   Val Thr Val Gly Leu Pro Ala Gly Ala Ser Phe Glu Tyr Lys Tyr Ile
   65                  70                  75                  80
   Arg Val Glu Ser Ser Gly Thr Ile Glu Trp Glu Ser Asp Pro Asn Arg
                   85                  90                  95
   Ser Tyr Thr Val Pro Ala Ala Cys Ala Thr Thr Ala Val Thr Glu Ser
               100                 105                 110
   Asp Thr
<210> 23
   <211> 111
   <212> PRT
   <213> Humicola grisea var. thermoidea
<400> 23
   Ala Asp Ala Ser Glu Val Tyr Val Thr Phe Asn Glu Arg Val Ser Thr
   1               5                   10                  15
   Ala Trp Gly Glu Thr Ile Lys Val Val Gly Asn Val Pro Ala Leu Gly
               20                  25                  30
   Asn Trp Asp Thr Ser Lys Ala Val Thr Leu Ser Ala Ser Gly Tyr Lys
           35                  40                  45
   Ser Asn Asp Pro Leu Trp Ser Ile Thr Val Pro Ile Lys Ala Thr Gly
       50                  55                  60
   Ser Ala Val Gln Tyr Lys Tyr Ile Lys Val Gly Thr Asn Gly Lys Ile
   65                  70                  75                  80
   Thr Trp Glu Ser Asp Pro Asn Arg Ser Ile Thr Leu Gln Thr Ala Ser
                   85                  90                  95
   Ser Ala Gly Lys Cys Ala Ala Gln Thr Val Asn Asp Ser Trp Arg
               100                 105                 110
<210> 24
   <211> 108
   <212> PRT
   <213> Aspergillus niger
<400> 24
<210> 25
   <211> 97
   <212> PRT
   <213> Aspergillus rolfsii
<400> 25
   Val Glu Val Thr Phe Asp Val Tyr Ala Thr Thr Val Tyr Gly Gln Asn
   1 5 10 15
   Ile Tyr Ile Thr Gly Asp Val Ser Glu Leu Gly Asn Trp Thr Pro Ala
   20 25 30
   Asn Gly Val Ala Leu Ser Ser Ala Asn Tyr Pro Thr Trp Ser Ala Thr
   35 40 45
   Ile Ala Leu Pro Ala Asp Thr Thr Ile Gln Tyr Lys Tyr Val Asn Ile
   50 55 60
   Asp Gly Ser Thr Val Ile Trp Glu Asp Ala Ile Ser Asn Arg Glu Ile
   65 70 75 80
   Thr Thr Pro Ala Ser Gly Thr Tyr Thr Glu Lys Asp Thr Trp Asp Glu
   85 90 95
   Ser
<210> 26
   <211> 38
   <212> PRT
   <213> Aspergillus niger
<400> 26
   Thr Gly Gly Thr Thr Thr Thr Ala Thr Pro Thr Gly Ser Gly Ser Val
   1 5 10 15
   Thr Ser Thr Ser Lys Thr Thr Ala Thr Ala Ser Lys Thr Ser Thr Ser
   20 25 30
   Thr Ser Ser Thr Ser Ala
   35
<210> 27
   <211> 31
   <212> PRT
   <213> Aspergillus kawachii
<400> 27
   Thr Thr Thr Thr Thr Thr Ala Ala Ala Thr Ser Thr Ser Lys Ala Thr
   1 5 10 15
   Thr Ser Ser Ser Ser Ser Ser Ala Ala Ala Thr Thr Ser Ser Ser
   20 25 30
<210> 28
   <211> 11
   <212> PRT
   <213> Athelia rolfsii
<400> 28
   Gly Ala Thr Ser Pro Gly Gly Ser Ser Gly Ser
   1               5                   10
<210> 29
   <211> 8
   <212> PRT
   <213> Artificial
<220>
   <223> Artificial
<400> 29
   Pro Glu Pro Thr Pro Glu Pro Thr
   1               5
<210> 30
   <211> 498
   <212> PRT
   <213> Aspergillus oryzae
<400> 30
   Met Val Ala Trp Trp Ser Leu Phe Leu Tyr Gly Leu Gln Val Ala Ala
   1               5                   10                  15
   Pro Ala Leu Ala Ala Thr Pro Ala Asp Trp Arg Ser Gln Ser Ile Tyr
               20                  25                  30
   Phe Leu Leu Thr Asp Arg Phe Ala Arg Thr Asp Gly Ser Thr Thr Ala
           35                  40                  45
   Thr Cys Asn Thr Ala Asp Gln Lys Tyr Cys Gly Gly Thr Trp Gln Gly
       50                  55                  60
   Ile Ile Asp Lys Leu Asp Tyr Ile Gln Gly Met Gly Phe Thr Ala Ile
   65                  70                  75                  80
   Trp Ile Thr Pro Val Thr Ala Gln Leu Pro Gln Thr Thr Ala Tyr Gly
                   85                  90                  95
   Asp Ala Tyr His Gly Tyr Trp Gln Gln Asp Ile Tyr Ser Leu Asn Glu
               100                 105                 110
   Asn Tyr Gly Thr Ala Asp Asp Leu Lys Ala Leu Ser Ser Ala Leu His
           115                 120                 125
   Glu Arg Gly Met Tyr Leu Met Val Asp Val Val Ala Asn His Met Gly
       130                 135                 140
   Tyr Asp Gly Ala Gly Ser Ser Val Asp Tyr Ser Val Phe Lys Pro Phe
   145                 150                 155                 160
   Ser Ser Gln Asp Tyr Phe His Pro Phe Cys Phe Ile Gln Asn Tyr Glu
                   165                 170                 175
   Asp Gln Thr Gln Val Glu Asp Cys Trp Leu Gly Asp Asn Thr Val Ser
               180                 185                 190
   Leu Pro Asp Leu Asp Thr Thr Lys Asp Val Val Lys Asn Glu Trp Tyr
           195                 200                 205
   Asp Trp Val Gly Ser Leu Val Ser Asn Tyr Ser Ile Asp Gly Leu Arg
       210                 215                 220
   Ile Asp Thr Val Lys His Val Gln Lys Asp Phe Trp Pro Gly Tyr Asn
   225                 230                 235                 240
   Lys Ala Ala Gly Val Tyr Cys Ile Gly Glu Val Leu Asp Gly Asp Pro
                   245                 250                 255
   Ala Tyr Thr Cys Pro Tyr Gln Asn Val Met Asp Gly Val Leu Asn Tyr
               260                 265                 270
   Pro Ile Tyr Tyr Pro Leu Leu Asn Ala Phe Lys Ser Thr Ser Gly Ser
           275                 280                 285
   Met Asp Asp Leu Tyr Asn Met Ile Asn Thr Val Lys Ser Asp Cys Pro
       290                 295                 300
   Asp Ser Thr Leu Leu Gly Thr Phe Val Glu Asn His Asp Asn Pro Arg
   305                 310                 315                 320
   Phe Ala Ser Tyr Thr Asn Asp Ile Ala Leu Ala Lys Asn Val Ala Ala
                   325                 330                 335
   Phe Ile Ile Leu Asn Asp Gly Ile Pro Ile Ile Tyr Ala Gly Gln Glu
               340                 345                 350
   Gln His Tyr Ala Gly Gly Asn Asp Pro Ala Asn Arg Glu Ala Thr Trp
           355                 360                 365
   Leu Ser Gly Tyr Pro Thr Asp Ser Glu Leu Tyr Lys Leu Ile Ala Ser
       370                 375                 380
   Ala Asn Ala Ile Arg Asn Tyr Ala Ile Ser Lys Asp Thr Gly Phe Val
   385                 390                 395                 400
   Thr Tyr Lys Asn Trp Pro Ile Tyr Lys Asp Asp Thr Thr Ile Ala Met
                   405                 410                 415
   Arg Lys Gly Thr Asp Gly Ser Gln Ile Val Thr Ile Leu Ser Asn Lys
               420                 425                 430
   Gly Ala Ser Gly Asp Ser Tyr Thr Leu Ser Leu Ser Gly Ala Gly Tyr
           435                 440                 445
   Thr Ala Gly Gln Gln Leu Thr Glu Val Ile Gly Cys Thr Thr Val Thr
       450                 455                 460
   Val Gly Ser Asp Gly Asn Val Pro Val Pro Met Ala Gly Gly Leu Pro
   465                 470                 475                 480
   Arg Val Leu Tyr Pro Thr Glu Lys Leu Ala Gly Ser Lys Ile Cys Ser
                   485                 490                 495
   Ser Ser
<210> 31
   <211> 1923
   <212> DNA
   <213> Artificial
<220>
   <223> Artificial
<220>
   <221> CDS
   <222> (1)..(1923)
<400> 31
   atg aga tta tcg act tcg agt ctc ttc ctt tcc gtg tct ctg ctg ggg      48
   Met Arg Leu Ser Thr Ser Ser Leu Phe Leu Ser Val Ser Leu Leu Gly
   1               5                   10                  15
   aag ctg gcc ctc ggg ctg tcg gct gca gaa tgg cgc act cag tcg att      96
   Lys Leu Ala Leu Gly Leu Ser Ala Ala Glu Trp Arg Thr Gln Ser Ile
               20                  25                  30
   tac ttc cta ttg acg gat cgg ttc ggt agg acg gac aat tcg acg aca     144
   Tyr Phe Leu Leu Thr Asp Arg Phe Gly Arg Thr Asp Asn Ser Thr Thr
           35                  40                  45
   gct aca tgc gat acg ggt gac caa atc tat tgt ggt ggc agt tgg caa      192
   Ala Thr Cys Asp Thr Gly Asp Gln Ile Tyr Cys Gly Gly Ser Trp Gln
       50                  55                  60
   gga atc atc aac cat ctg gat tat atc cag ggc atg gga ttc acg gcc      240
   Gly Ile Ile Asn His Leu Asp Tyr Ile Gln Gly Met Gly Phe Thr Ala
   65                  70                  75                  80
   atc tgg atc tcg cct atc act gaa cag ctg ccc cag gat act gct gat      288
   Ile Trp Ile Ser Pro Ile Thr Glu Gln Leu Pro Gln Asp Thr Ala Asp
                   85                  90                  95
   ggt gaa gct tac cat gga tat tgg cag cag aag ata tac gac gtg aac      336
   Gly Glu Ala Tyr His Gly Tyr Trp Gln Gln Lys Ile Tyr Asp Val Asn
               100                 105                 110
   tcc aac ttc ggc act gca gat gac ctc aag tcc ctc tca gat gcg ctt      384
   Ser Asn Phe Gly Thr Ala Asp Asp Leu Lys Ser Leu Ser Asp Ala Leu
           115                 120                 125
   cat gcc cgc gga atg tac ctc atg gtg gac gtc gtc cct aac cac atg      432
   His Ala Arg Gly Met Tyr Leu Met Val Asp Val Val Pro Asn His Met
       130                 135                 140
   ggc tac gcc ggc aac ggc aac gat gta gac tac agc gtc ttc gac ccc      480
   Gly Tyr Ala Gly Asn Gly Asn Asp Val Asp Tyr Ser Val Phe Asp Pro
   145                 150                 155                 160
   ttc gat tcc tcc tcc tac ttc cac cca tac tgc ctg atc aca gat tgg      528
   Phe Asp Ser Ser Ser Tyr Phe His Pro Tyr Cys Leu Ile Thr Asp Trp
                   165                 170                 175
   gac aac ttg acc atg gtc caa gat tgt tgg gag ggt gac acc atc gta      576
   Asp Asn Leu Thr Met Val Gln Asp Cys Trp Glu Gly Asp Thr Ile Val
               180                 185                 190
   tct ctg cca gac cta aac acc acc gaa act gcc gtg aga aca atc tgg      624
   Ser Leu Pro Asp Leu Asn Thr Thr Glu Thr Ala Val Arg Thr Ile Trp
           195                 200                 205
   tat gac tgg gta gcc gac ctg gta tcc aat tat tca gtc gac gga ctc      672
   Tyr Asp Trp Val Ala Asp Leu Val Ser Asn Tyr Ser Val Asp Gly Leu
       210                 215                 220
   cgc atc gac agt gtc ctc gaa gtc gaa cca gac ttc ttc ccg ggc tac      720
   Arg Ile Asp Ser Val Leu Glu Val Glu Pro Asp Phe Phe Pro Gly Tyr
   225                 230                 235                 240
   cag gaa gca gca ggt gtc tac tgc gtc ggc gaa gtc gac aac ggc aac      768
   Gln Glu Ala Ala Gly Val Tyr Cys Val Gly Glu Val Asp Asn Gly Asn
                   245                 250                 255
   cct gcc ctc gac tgc cca tac cag aag gtc ctg gac ggc gtc ctc aac      816
   Pro Ala Leu Asp Cys Pro Tyr Gln Lys Val Leu Asp Gly Val Leu Asn
               260                 265                 270
   tat ccg atc tac tgg caa ctc ctc tac gcc ttc gaa tcc tcc agc ggc      864
   Tyr Pro Ile Tyr Trp Gln Leu Leu Tyr Ala Phe Glu Ser Ser Ser Gly
           275                 280                 285
   agc atc agc aat ctc tac aac atg atc aaa tcc gtc gca agc gac tgc      912
   Ser Ile Ser Asn Leu Tyr Asn Met Ile Lys Ser Val Ala Ser Asp Cys
       290                 295                 300
   tcc gat ccg aca cta ctc ggc aac ttc atc gaa aac cac gac aat ccc      960
   Ser Asp Pro Thr Leu Leu Gly Asn Phe Ile Glu Asn His Asp Asn Pro
   305                 310                 315                 320
   cgt ttc gcc tcc tac acc tcc gac tac tcg caa gcc aaa aac gtc ctc     1008
   Arg Phe Ala Ser Tyr Thr Ser Asp Tyr Ser Gln Ala Lys Asn Val Leu
                   325                 330                 335
   agc tac atc ttc ctc tcc gac ggc atc ccc atc gtc tac gcc ggc gaa     1056
   Ser Tyr Ile Phe Leu Ser Asp Gly Ile Pro Ile Val Tyr Ala Gly Glu
               340                 345                 350
   gaa cag cac tac tcc ggc ggc aag gtg ccc tac aac cgc gaa gcg acc     1104
   Glu Gln His Tyr Ser Gly Gly Lys Val Pro Tyr Asn Arg Glu Ala Thr
           355                 360                 365
   tgg ctt tca ggc tac gac acc tcc gca gag ctg tac acc tgg ata gcc     1152
   Trp Leu Ser Gly Tyr Asp Thr Ser Ala Glu Leu Tyr Thr Trp Ile Ala
       370                 375                 380
   acc acg aac gcg atc cgc aaa cta gcc atc tca gct gac tcg gcc tac     1200
   Thr Thr Asn Ala Ile Arg Lys Leu Ala Ile Ser Ala Asp Ser Ala Tyr
   385                 390                 395                 400
   att acc tac gcg aat gat gca ttc tac act gac agc aac acc atc gca     1248
   Ile Thr Tyr Ala Asn Asp Ala Phe Tyr Thr Asp Ser Asn Thr Ile Ala
                   405                 410                 415
   atg cgc aaa ggc acc tca ggg agc caa gtc atc acc gtc ctc tcc aac     1296
   Met Arg Lys Gly Thr Ser Gly Ser Gln Val Ile Thr Val Leu Ser Asn
               420                 425                 430
   aaa ggc tcc tca gga agc agc tac acc ctg acc ctc agc gga agc ggc     1344
   Lys Gly Ser Ser Gly Ser Ser Tyr Thr Leu Thr Leu Ser Gly Ser Gly
           435                 440                 445
   tac aca tcc ggc acg aag ctg atc gaa gcg tac aca tgc aca tcc gtg     1392
   Tyr Thr Ser Gly Thr Lys Leu Ile Glu Ala Tyr Thr Cys Thr Ser Val
       450                 455                 460
   acc gtg gac tcg agc ggc gat att ccc gtg ccg atg gcg tcg gga tta     1440
   Thr Val Asp Ser Ser Gly Asp Ile Pro Val Pro Met Ala Ser Gly Leu
   465                 470                 475                 480
   ccg aga gtt ctt ctg ccc gcg tcc gtc gtc gat agc tct tcg ctc tgt     1488
   Pro Arg Val Leu Leu Pro Ala Ser Val Val Asp Ser Ser Ser Leu Cys
                   485                 490                 495
   ggc ggg agc gga aga aca acc acg acc aca act gct gct gct act agt     1536
   Gly Gly Ser Gly Arg Thr Thr Thr Thr Thr Thr Ala Ala Ala Thr Ser
               500                 505                 510
   aca tcc aaa gcc acc acc tcc tct tct tct tct tct gct gct gct act     1584
   Thr Ser Lys Ala Thr Thr Ser Ser Ser Ser Ser Ser Ala Ala Ala Thr
           515                 520                 525
   act tct tca tca tgt acc act ccc acc gcc gtg gct gtg act ttc gat     1632
   Thr Ser Ser Ser Cys Thr Thr Pro Thr Ala Val Ala Val Thr Phe Asp
       530                 535                 540
   ctg aca gct acc acc acc tac ggc gag aac atc tac ctg gtc gga tcg      1680
   Leu Thr Ala Thr Thr Thr Tyr Gly Glu Asn Ile Tyr Leu Val Gly Ser
   545                 550                 555                 560
   atc tct cag ctg ggt gac tgg gaa acc agc gac ggc ata gct ctg agt      1728
   Ile Ser Gln Leu Gly Asp Trp Glu Thr Ser Asp Gly Ile Ala Leu Ser
                   565                 570                 575
   gct gac aag tac act tcc agc gac ccg ctc tgg tat gtc act gtg act      1776
   Ala Asp Lys Tyr Thr Ser Ser Asp Pro Leu Trp Tyr Val Thr Val Thr
               580                 585                 590
   ctg ccg gct ggt gag tcg ttt gag tac aag ttt atc cgc att gag agc      1824
   Leu Pro Ala Gly Glu Ser Phe Glu Tyr Lys Phe Ile Arg Ile Glu Ser
           595                 600                 605
   gat gac tcc gtg gag tgg gag agt gat ccc aac cga gaa tac acc gtt      1872
   Asp Asp Ser Val Glu Trp Glu Ser Asp Pro Asn Arg Glu Tyr Thr Val
       610                 615                 620
   cct cag gcg tgc gga acg tcg acc gcg acg gtg act gac acc tgg cgg      1920
   Pro Gln Ala Cys Gly Thr Ser Thr Ala Thr Val Thr Asp Thr Trp Arg
   625                 630                 635                 640
   tag                                                                  1923
<210> 32
   <211> 640

   <212> PRT <213> Artificial
<220>
   <223> Artificial
<400> 32
   Met Arg Leu Ser Thr Ser Ser Leu Phe Leu Ser Val Ser Leu Leu Gly
   1               5                   10                  15
   Lys Leu Ala Leu Gly Leu Ser Ala Ala Glu Trp Arg Thr Gln Ser Ile
               20                  25                  30
   Tyr Phe Leu Leu Thr Asp Arg Phe Gly Arg Thr Asp Asn Ser Thr Thr
           35                  40                  45
   Ala Thr Cys Asp Thr Gly Asp Gln Ile Tyr Cys Gly Gly Ser Trp Gln
       50                  55                  60
   Gly Ile Ile Asn His Leu Asp Tyr Ile Gln Gly Met Gly Phe Thr Ala
   65                  70                  75                  80
   Ile Trp Ile Ser Pro Ile Thr Glu Gln Leu Pro Gln Asp Thr Ala Asp
   85 90 95
   Gly Glu Ala Tyr His Gly Tyr Trp Gln Gln Lys Ile Tyr Asp Val Asn
   100 105 110
   Ser Asn Phe Gly Thr Ala Asp Asp Leu Lys Ser Leu Ser Asp Ala Leu
   115 120 125
   His Ala Arg Gly Met Tyr Leu Met Val Asp Val Val Pro Asn His Met
   130 135 140
   Gly Tyr Ala Gly Asn Gly Asn Asp Val Asp Tyr Ser Val Phe Asp Pro
   145 150 155 160
   Phe Asp Ser Ser Ser Tyr Phe His Pro Tyr Cys Leu Ile Thr Asp Trp
   165 170 175
   Asp Asn Leu Thr Met Val Gln Asp Cys Trp Glu Gly Asp Thr Ile Val
   180 185 190
   Ser Leu Pro Asp Leu Asn Thr Thr Glu Thr Ala Val Arg Thr Ile Trp
   195 200 205
   Tyr Asp Trp Val Ala Asp Leu Val Ser Asn Tyr Ser Val Asp Gly Leu
   210 215 220
   Arg Ile Asp Ser Val Leu Glu Val Glu Pro Asp Phe Phe Pro Gly Tyr
   225 230 235 240
   Gln Glu Ala Ala Gly Val Tyr Cys Val Gly Glu Val Asp Asn Gly Asn
   245 250 255
   Pro Ala Leu Asp Cys Pro Tyr Gln Lys Val Leu Asp Gly Val Leu Asn
   260 265 270
   Tyr Pro Ile Tyr Trp Gln Leu Leu Tyr Ala Phe Glu Ser Ser Ser Gly
   275 280 285
   Ser Ile Ser Asn Leu Tyr Asn Met Ile Lys Ser Val Ala Ser Asp Cys
   290 295 300
   Ser Asp Pro Thr Leu Leu Gly Asn Phe Ile Glu Asn His Asp Asn Pro
   305 310 315 320
   Arg Phe Ala Ser Tyr Thr Ser Asp Tyr Ser Gln Ala Lys Asn Val Leu
   325 330 335
   Ser Tyr Ile Phe Leu Ser Asp Gly Ile Pro Ile Val Tyr Ala Gly Glu
                   340                 345                 350
   Glu Gln His Tyr Ser Gly Gly Lys Val Pro Tyr Asn Arg Glu Ala Thr
           355                 360                 365
   Trp Leu Ser Gly Tyr Asp Thr Ser Ala Glu Leu Tyr Thr Trp Ile Ala
       370                 375                 380
   Thr Thr Asn Ala Ile Arg Lys Leu Ala Ile Ser Ala Asp Ser Ala Tyr
   385                 390                 395                 400
   Ile Thr Tyr Ala Asn Asp Ala Phe Tyr Thr Asp Ser Asn Thr Ile Ala
                   405                 410                 415
   Met Arg Lys Gly Thr Ser Gly Ser Gln Val Ile Thr Val Leu Ser Asn
               420                 425                 430
   Lys Gly Ser Ser Gly Ser Ser Tyr Thr Leu Thr Leu Ser Gly Ser Gly
           435                 440                 445
   Tyr Thr Ser Gly Thr Lys Leu Ile Glu Ala Tyr Thr Cys Thr Ser Val
       450                 455                 460
   Thr Val Asp Ser Ser Gly Asp Ile Pro Val Pro Met Ala Ser Gly Leu
   465                 470                 475                 480
   Pro Arg Val Leu Leu Pro Ala Ser Val Val Asp Ser Ser Ser Leu Cys
                   485                 490                 495
   Gly Gly Ser Gly Arg Thr Thr Thr Thr Thr Thr Ala Ala Ala Thr Ser
               500                 505                 510
   Thr Ser Lys Ala Thr Thr Ser Ser Ser Ser Ser Ser Ala Ala Ala Thr
           515                 520                 525
   Thr Ser Ser Ser Cys Thr Thr Pro Thr Ala Val Ala Val Thr Phe Asp
       530                 535                 540
   Leu Thr Ala Thr Thr Thr Tyr Gly Glu Asn Ile Tyr Leu Val Gly Ser
   545                 550                 555                 560
   Ile Ser Gln Leu Gly Asp Trp Glu Thr Ser Asp Gly Ile Ala Leu Ser
                   565                 570                 575
   Ala Asp Lys Tyr Thr Ser Ser Asp Pro Leu Trp Tyr Val Thr Val Thr
               580                 585                 590
   Leu Pro Ala Gly Glu Ser Phe Glu Tyr Lys Phe Ile Arg Ile Glu Ser
           595                 600                 605
   Asp Asp Ser Val Glu Trp Glu Ser Asp Pro Asn Arg Glu Tyr Thr Val
       610                 615                 620
   Pro Gln Ala Cys Gly Thr Ser Thr Ala Thr Val Thr Asp Thr Trp Arg
   625                 630                 635                 640
<210> 33
   <211> 1890
   <212> DNA
   <213> Artificial
<220>
   <223> Artificial
<220>
   <221> CDS
   <222> (1)..(1890)
<400> 33
   atg aga tta tcg act tcg agt ctc ttc ctt tcc gtg tct ctg ctg ggg      48
   Met Arg Leu Ser Thr Ser Ser Leu Phe Leu Ser Val Ser Leu Leu Gly
   1               5                   10                  15
   aag ctg gcc ctc ggg ctg tcg gct gca gaa tgg cgc act cag tcg att      96
   Lys Leu Ala Leu Gly Leu Ser Ala Ala Glu Trp Arg Thr Gln Ser Ile
               20                  25                  30
   tac ttc cta ttg acg gat cgg ttc ggt agg acg gac aat tcg acg aca     144
   Tyr Phe Leu Leu Thr Asp Arg Phe Gly Arg Thr Asp Asn Ser Thr Thr
           35                  40                  45
   gct aca tgc gat acg ggt gac caa atc tat tgt ggt ggc agt tgg caa     192
   Ala Thr Cys Asp Thr Gly Asp Gln Ile Tyr Cys Gly Gly Ser Trp Gln
       50                  55                  60
   gga atc atc aac cat ctg gat tat atc cag ggc atg gga ttc acg gcc     240
   Gly Ile Ile Asn His Leu Asp Tyr Ile Gln Gly Met Gly Phe Thr Ala
   65                  70                  75                  80
   atc tgg atc tcg cct atc act gaa cag ctg ccc cag gat act gct gat     288
   Ile Trp Ile Ser Pro Ile Thr Glu Gln Leu Pro Gln Asp Thr Ala Asp
                   85                  90                  95
   ggt gaa gct tac cat gga tat tgg cag cag aag ata tac gac gtg aac     336
   Gly Glu Ala Tyr His Gly Tyr Trp Gln Gln Lys Ile Tyr Asp Val Asn
               100                 105                 110
   tcc aac ttc ggc act gca gat gac ctc aag tcc ctc tca gat gcg ctt     384
   Ser Asn Phe Gly Thr Ala Asp Asp Leu Lys Ser Leu Ser Asp Ala Leu
           115                 120                 125
   cat gcc cgc gga atg tac ctc atg gtg gac gtc gtc cct aac cac atg     432
   His Ala Arg Gly Met Tyr Leu Met Val Asp Val Val Pro Asn His Met
       130                 135                 140
   ggc tac gcc ggc aac ggc aac gat gta gac tac agc gtc ttc gac ccc     480
   Gly Tyr Ala Gly Asn Gly Asn Asp Val Asp Tyr Ser Val Phe Asp Pro
   145                 150                 155                 160
   ttc gat tcc tcc tcc tac ttc cac cca tac tgc ctg atc aca gat tgg     528
   Phe Asp Ser Ser Ser Tyr Phe His Pro Tyr Cys Leu Ile Thr Asp Trp
                   165                 170                 175
   gac aac ttg acc atg gtc caa gat tgt tgg gag ggt gac acc atc gta     576
   Asp Asn Leu Thr Met Val Gln Asp Cys Trp Glu Gly Asp Thr Ile Val
               180                 185                 190
   tct ctg cca gac cta aac acc acc gaa act gcc gtg aga aca atc tgg     624
   Ser Leu Pro Asp Leu Asn Thr Thr Glu Thr Ala Val Arg Thr Ile Trp
           195                 200                 205
   tat gac tgg gta gcc gac ctg gta tcc aat tat tca gtc gac gga ctc     672
   Tyr Asp Trp Val Ala Asp Leu Val Ser Asn Tyr Ser Val Asp Gly Leu
       210                 215                 220
   cgc atc gac agt gtc ctc gaa gtc gaa cca gac ttc ttc ccg ggc tac     720
   Arg Ile Asp Ser Val Leu Glu Val Glu Pro Asp Phe Phe Pro Gly Tyr
   225                 230                 235                 240
   cag gaa gca gca ggt gtc tac tgc gtc ggc gaa gtc gac aac ggc aac     768
   Gln Glu Ala Ala Gly Val Tyr Cys Val Gly Glu Val Asp Asn Gly Asn
                   245                 250                 255
   cct gcc ctc gac tgc cca tac cag aag gtc ctg gac ggc gtc ctc aac     816
   Pro Ala Leu Asp Cys Pro Tyr Gln Lys Val Leu Asp Gly Val Leu Asn
               260                 265                 270
   tat ccg atc tac tgg caa ctc ctc tac gcc ttc gaa tcc tcc agc ggc     864
   Tyr Pro Ile Tyr Trp Gln Leu Leu Tyr Ala Phe Glu Ser Ser Ser Gly
           275                 280                 285
   agc atc agc aat ctc tac aac atg atc aaa tcc gtc gca agc gac tgc     912
   Ser Ile Ser Asn Leu Tyr Asn Met Ile Lys Ser Val Ala Ser Asp Cys
       290                 295                 300
   tcc gat ccg aca cta ctc ggc aac ttc atc gaa aac cac gac aat ccc     960
   Ser Asp Pro Thr Leu Leu Gly Asn Phe Ile Glu Asn His Asp Asn Pro
   305                 310                 315                 320
   cgt ttc gcc tcc tac acc tcc gac tac tcg caa gcc aaa aac gtc ctc    1008
   Arg Phe Ala Ser Tyr Thr Ser Asp Tyr Ser Gln Ala Lys Asn Val Leu
                   325                 330                 335
   agc tac atc ttc ctc tcc gac ggc atc ccc atc gtc tac gcc ggc gaa    1056
   Ser Tyr Ile Phe Leu Ser Asp Gly Ile Pro Ile Val Tyr Ala Gly Glu
               340                 345                 350
   gaa cag cac tac tcc ggc ggc aag gtg ccc tac aac cgc gaa gcg acc    1104
   Glu Gln His Tyr Ser Gly Gly Lys Val Pro Tyr Asn Arg Glu Ala Thr
           355                 360                 365
   tgg ctt tca ggc tac gac acc tcc gca gag ctg tac acc tgg ata gcc      1152
   Trp Leu Ser Gly Tyr Asp Thr Ser Ala Glu Leu Tyr Thr Trp Ile Ala
       370                 375                 380
   acc acg aac gcg atc cgc aaa cta gcc atc tca gct gac tcg gcc tac      1200
   Thr Thr Asn Ala Ile Arg Lys Leu Ala Ile Ser Ala Asp Ser Ala Tyr
   385                 390                 395                 400
   att acc tac gcg aat gat gca ttc tac act gac agc aac acc atc gca      1248
   Ile Thr Tyr Ala Asn Asp Ala Phe Tyr Thr Asp Ser Asn Thr Ile Ala
                   405                 410                 415
   atg cgc aaa ggc acc tca ggg agc caa gtc atc acc gtc ctc tcc aac      1296
   Met Arg Lys Gly Thr Ser Gly Ser Gln Val Ile Thr Val Leu Ser Asn
               420                 425                 430
   aaa ggc tcc tca gga agc agc tac acc ctg acc ctc agc gga agc ggc      1344
   Lys Gly Ser Ser Gly Ser Ser Tyr Thr Leu Thr Leu Ser Gly Ser Gly
           435                 440                 445
   tac aca tcc ggc acg aag ctg atc gaa gcg tac aca tgc aca tcc gtg      1392
   Tyr Thr Ser Gly Thr Lys Leu Ile Glu Ala Tyr Thr Cys Thr Ser Val
       450                 455                 460
   acc gtg gac tcg agc ggc gat att ccc gtg ccg atg gcg tcg gga tta      1440
   Thr Val Asp Ser Ser Gly Asp Ile Pro Val Pro Met Ala Ser Gly Leu
   465                 470                 475                 480
   ccg aga gtt ctt ctg ccc gcg tcc gtc gtc gat agc tct tcg ctc tgt      1488
   Pro Arg Val Leu Leu Pro Ala Ser Val Val Asp Ser Ser Ser Leu Cys
                   485                 490                 495
   ggc ggg agc gga aga aca acc acg acc aca act gct gct gct act agt      1536
   Gly Gly Ser Gly Arg Thr Thr Thr Thr Thr Thr Ala Ala Ala Thr Ser
               500                 505                 510
   aca tcc aaa gcc acc acc tcc tct tct tct tct tct gct gct gct act      1584
   Thr Ser Lys Ala Thr Thr Ser Ser Ser Ser Ser Ser Ala Ala Ala Thr
           515                 520                 525
   act tct tca tca gtc gag gtc act ttc gac gtt tac gct acc aca gta      1632
   Thr Ser Ser Ser Val Glu Val Thr Phe Asp Val Tyr Ala Thr Thr Val
       530                 535                 540
   tat ggc cag aac atc tat atc acc ggt gat gtg agt gag ctc ggc aac      1680
   Tyr Gly Gln Asn Ile Tyr Ile Thr Gly Asp Val Ser Glu Leu Gly Asn
   545                 550                 555                 560
   tgg aca ccc gcc aat ggt gtt gca ctc tct tct gct aac tac ccc acc      1728
   Trp Thr Pro Ala Asn Gly Val Ala Leu Ser Ser Ala Asn Tyr Pro Thr
                   565                 570                 575
   tgg agt gcc acg atc gct ctc ccc gct gac acg aca atc cag tac aag      1776
   Trp Ser Ala Thr Ile Ala Leu Pro Ala Asp Thr Thr Ile Gln Tyr Lys
               580                 585                 590
   tat gtc aac att gac ggc agc acc gtc atc tgg gag gat gct atc agc      1824
   Tyr Val Asn Ile Asp Gly Ser Thr Val Ile Trp Glu Asp Ala Ile Ser
           595                 600                 605
   aat cgc gag atc acg acg ccc gcc agc ggc aca tac acc gaa aaa gac      1872
   Asn Arg Glu Ile Thr Thr Pro Ala Ser Gly Thr Tyr Thr Glu Lys Asp
       610                 615                 620
   act tgg gat gaa tct tag                                              1890
   Thr Trp Asp Glu Ser
   625
<210> 34
   <211> 629
   <212> PRT
   <213> Artificial
<220>
   <223> Artificial
<400> 34
   Met Arg Leu Ser Thr Ser Ser Leu Phe Leu Ser Val Ser Leu Leu Gly
   1               5                   10                  15
   Lys Leu Ala Leu Gly Leu Ser Ala Ala Glu Trp Arg Thr Gln Ser Ile
               20                  25                  30
   Tyr Phe Leu Leu Thr Asp Arg Phe Gly Arg Thr Asp Asn Ser Thr Thr
           35                  40                  45
   Ala Thr Cys Asp Thr Gly Asp Gln Ile Tyr Cys Gly Gly Ser Trp Gln
       50                  55                  60
   Gly Ile Ile Asn His Leu Asp Tyr Ile Gln Gly Met Gly Phe Thr Ala
   65                  70                  75                  80
   Ile Trp Ile Ser Pro Ile Thr Glu Gln Leu Pro Gln Asp Thr Ala Asp
                   85                  90                  95
   Gly Glu Ala Tyr His Gly Tyr Trp Gln Gln Lys Ile Tyr Asp Val Asn
               100                 105                 110
   Ser Asn Phe Gly Thr Ala Asp Asp Leu Lys Ser Leu Ser Asp Ala Leu
           115                 120                 125
   His Ala Arg Gly Met Tyr Leu Met Val Asp Val Val Pro Asn His Met
       130                 135                 140
   Gly Tyr Ala Gly Asn Gly Asn Asp Val Asp Tyr Ser Val Phe Asp Pro
   145                 150                 155                 160
   Phe Asp Ser Ser Ser Tyr Phe His Pro Tyr Cys Leu Ile Thr Asp Trp
                   165                 170                 175
   Asp Asn Leu Thr Met Val Gln Asp Cys Trp Glu Gly Asp Thr Ile Val
               180                 185                 190
   Ser Leu Pro Asp Leu Asn Thr Thr Glu Thr Ala Val Arg Thr Ile Trp
           195                 200                 205
   Tyr Asp Trp Val Ala Asp Leu Val Ser Asn Tyr Ser Val Asp Gly Leu
       210                 215                 220
   Arg Ile Asp Ser Val Leu Glu Val Glu Pro Asp Phe Phe Pro Gly Tyr
   225                 230                 235                 240
   Gln Glu Ala Ala Gly Val Tyr Cys Val Gly Glu Val Asp Asn Gly Asn
                   245                 250                 255
   Pro Ala Leu Asp Cys Pro Tyr Gln Lys Val Leu Asp Gly Val Leu Asn
               260                 265                 270
   Tyr Pro Ile Tyr Trp Gln Leu Leu Tyr Ala Phe Glu Ser Ser Ser Gly
           275                 280                 285
   Ser Ile Ser Asn Leu Tyr Asn Met Ile Lys Ser Val Ala Ser Asp Cys
       290                 295                 300
   Ser Asp Pro Thr Leu Leu Gly Asn Phe Ile Glu Asn His Asp Asn Pro
   305                 310                 315                 320
   Arg Phe Ala Ser Tyr Thr Ser Asp Tyr Ser Gln Ala Lys Asn Val Leu
                   325                 330                 335
   Ser Tyr Ile Phe Leu Ser Asp Gly Ile Pro Ile Val Tyr Ala Gly Glu
               340                 345                 350
   Glu Gln His Tyr Ser Gly Gly Lys Val Pro Tyr Asn Arg Glu Ala Thr
           355                 360                 365
   Trp Leu Ser Gly Tyr Asp Thr Ser Ala Glu Leu Tyr Thr Trp Ile Ala
       370                 375                 380
   Thr Thr Asn Ala Ile Arg Lys Leu Ala Ile Ser Ala Asp Ser Ala Tyr
   385                 390                 395                 400
   Ile Thr Tyr Ala Asn Asp Ala Phe Tyr Thr Asp Ser Asn Thr Ile Ala
                   405                 410                 415
   Met Arg Lys Gly Thr Ser Gly Ser Gln Val Ile Thr Val Leu Ser Asn
               420                 425                 430
   Lys Gly Ser Ser Gly Ser Ser Tyr Thr Leu Thr Leu Ser Gly Ser Gly
           435                 440                 445
   Tyr Thr Ser Gly Thr Lys Leu Ile Glu Ala Tyr Thr Cys Thr Ser Val
       450                 455                 460
   Thr Val Asp Ser Ser Gly Asp Ile Pro Val Pro Met Ala Ser Gly Leu
   465                 470                 475                 480
   Pro Arg Val Leu Leu Pro Ala Ser Val Val Asp Ser Ser Ser Leu Cys
                   485                 490                 495
   Gly Gly Ser Gly Arg Thr Thr Thr Thr Thr Thr Ala Ala Ala Thr Ser
               500                 505                 510
   Thr Ser Lys Ala Thr Thr Ser Ser Ser Ser Ser Ser Ala Ala Ala Thr
           515                 520                 525
   Thr Ser Ser Ser Val Glu Val Thr Phe Asp Val Tyr Ala Thr Thr Val
       530                 535                 540
   Tyr Gly Gln Asn Ile Tyr Ile Thr Gly Asp Val Ser Glu Leu Gly Asn
   545                 550                 555                 560
   Trp Thr Pro Ala Asn Gly Val Ala Leu Ser Ser Ala Asn Tyr Pro Thr
                   565                 570                 575
   Trp Ser Ala Thr Ile Ala Leu Pro Ala Asp Thr Thr Ile Gln Tyr Lys
               580                 585                 590
   Tyr Val Asn Ile Asp Gly Ser Thr Val Ile Trp Glu Asp Ala Ile Ser
           595                 600                 605
   Asn Arg Glu Ile Thr Thr Pro Ala Ser Gly Thr Tyr Thr Glu Lys Asp
       610                 615                 620
   Thr Trp Asp Glu Ser
   625
<210> 35
   <211> 1923
   <212> DNA
   <213> Artificial
<220>
   <223> Artificial
<220>
   <221> CDS
   <222> (1)..(1923)
<400> 35
   atg gtc gcg tgg tgg tct cta ttt ctg tac ggc ctt cag gtc gcg gca       48
   Met Val Ala Trp Trp Ser Leu Phe Leu Tyr Gly Leu Gln Val Ala Ala
   1               5                   10                  15
   cct gct ttg gct gca acg cct gcg gac tgg cga tcg caa tcc att tat       96
   Pro Ala Leu Ala Ala Thr Pro Ala Asp Trp Arg Ser Gln Ser Ile Tyr
               20                  25                  30
   ttc ctt ctc acg gat cga ttt gca agg acg gat ggg tcg acg act gcg      144
   Phe Leu Leu Thr Asp Arg Phe Ala Arg Thr Asp Gly Ser Thr Thr Ala
           35                  40                  45
   act tgt aat act gcg gat cag aaa tac tgt ggt gga aca tgg cag ggc      192
   Thr Cys Asn Thr Ala Asp Gln Lys Tyr Cys Gly Gly Thr Trp Gln Gly
       50                  55                  60
   atc atc gac aag ttg gac tat atc cag gga atg ggc ttc aca gcc atc      240
   Ile Ile Asp Lys Leu Asp Tyr Ile Gln Gly Met Gly Phe Thr Ala Ile
   65                  70                  75                  80
   tgg atc acc ccc gtt aca gcc cag ctg ccc cag acc acc gca tat gga      288
   Trp Ile Thr Pro Val Thr Ala Gln Leu Pro Gln Thr Thr Ala Tyr Gly
                   85                  90                  95
   gat gcc tac cat ggc tac tgg cag cag gat ata tac tct ctg aac gaa      336
   Asp Ala Tyr His Gly Tyr Trp Gln Gln Asp Ile Tyr Ser Leu Asn Glu
               100                 105                 110
   aac tac ggc act gca gat gac ttg aag gcg ctc tct tcg gcc ctt cat      384
   Asn Tyr Gly Thr Ala Asp Asp Leu Lys Ala Leu Ser Ser Ala Leu His
           115                 120                 125
   gag agg ggg atg tat ctt atg gtc gat gtg gtt gct aac cat atg ggc      432
   Glu Arg Gly Met Tyr Leu Met Val Asp Val Val Ala Asn His Met Gly
       130                 135                 140
   tat gat gga gcg ggt agc tca gtc gat tac agt gtg ttt aaa ccg ttc      480
   Tyr Asp Gly Ala Gly Ser Ser Val Asp Tyr Ser Val Phe Lys Pro Phe
   145                 150                 155                 160
   agt tcc caa gac tac ttc cac ccg ttc tgt ttc att caa aac tat gaa      528
   Ser Ser Gln Asp Tyr Phe His Pro Phe Cys Phe Ile Gln Asn Tyr Glu
                   165                 170                 175
   gat cag act cag gtt gag gat tgc tgg cta gga gat aac act gtc tcc      576
   Asp Gln Thr Gln Val Glu Asp Cys Trp Leu Gly Asp Asn Thr Val Ser
               180                 185                 190
   ttg cct gat ctc gat acc acc aag gat gtg gtc aag aat gaa tgg tac      624
   Leu Pro Asp Leu Asp Thr Thr Lys Asp Val Val Lys Asn Glu Trp Tyr
           195                 200                 205
   gac tgg gtg gga tca ttg gta tcg aac tac tcc att gac ggc ctc cgt      672
   Asp Trp Val Gly Ser Leu Val Ser Asn Tyr Ser Ile Asp Gly Leu Arg
       210                 215                 220
   atc gac aca gta aaa cac gtc cag aag gac ttc tgg ccc ggg tac aac      720
   Ile Asp Thr Val Lys His Val Gln Lys Asp Phe Trp Pro Gly Tyr Asn
   225                 230                 235                 240
   aaa gcc gca ggc gtg tac tgt atc ggc gag gtg ctc gac ggt gat ccg      768
   Lys Ala Ala Gly Val Tyr Cys Ile Gly Glu Val Leu Asp Gly Asp Pro
                   245                 250                 255
   gcc tac act tgt ccc tac cag aac gtc atg gac ggc gta ctg aac tat      816
   Ala Tyr Thr Cys Pro Tyr Gln Asn Val Met Asp Gly Val Leu Asn Tyr
               260                 265                 270
   ccc att tac tat cca ctc ctc aac gcc ttc aag tca acc tcc ggc agc      864
   Pro Ile Tyr Tyr Pro Leu Leu Asn Ala Phe Lys Ser Thr Ser Gly Ser
           275                 280                 285
   atg gac gac ctc tac aac atg atc aac acc gtc aaa tcc gac tgt cca      912
   Met Asp Asp Leu Tyr Asn Met Ile Asn Thr Val Lys Ser Asp Cys Pro
       290                 295                 300
   gac tca aca ctc ctg ggc aca ttc gtc gag aac cac gac aac cca cgg      960
   Asp Ser Thr Leu Leu Gly Thr Phe Val Glu Asn His Asp Asn Pro Arg
   305                 310                 315                 320
   ttc gct tct tac acc aac gac ata gcc ctc gcc aag aac gtc gca gca     1008
   Phe Ala Ser Tyr Thr Asn Asp Ile Ala Leu Ala Lys Asn Val Ala Ala
                   325                 330                 335
   ttc atc atc ctc aac gac gga atc ccc atc atc tac gcc ggc caa gaa     1056
   Phe Ile Ile Leu Asn Asp Gly Ile Pro Ile Ile Tyr Ala Gly Gln Glu
               340                 345                 350
   cag cac tac gcc ggc gga aac gac ccc gcg aac cgc gaa gca acc tgg     1104
   Gln His Tyr Ala Gly Gly Asn Asp Pro Ala Asn Arg Glu Ala Thr Trp
           355                 360                 365
   ctc tcg ggc tac ccg acc gac agc gag ctg tac aag tta att gcc tcc     1152
   Leu Ser Gly Tyr Pro Thr Asp Ser Glu Leu Tyr Lys Leu Ile Ala Ser
       370                 375                 380
   gcg aac gca atc cgg aac tat gcc att agc aaa gat aca gga ttc gtg     1200
   Ala Asn Ala Ile Arg Asn Tyr Ala Ile Ser Lys Asp Thr Gly Phe Val
   385                 390                 395                 400
   acc tac aag aac tgg ccc atc tac aaa gac gac aca acg atc gcc atg     1248
   Thr Tyr Lys Asn Trp Pro Ile Tyr Lys Asp Asp Thr Thr Ile Ala Met
                   405                 410                 415
   cgc aag ggc aca gat ggg tcg cag atc gtg act atc ttg tcc aac aag     1296
   Arg Lys Gly Thr Asp Gly Ser Gln Ile Val Thr Ile Leu Ser Asn Lys
               420                 425                 430
   ggt gct tcg ggt gat tcg tat acc ctc tcc ttg agt ggt gcg ggt tac     1344
   Gly Ala Ser Gly Asp Ser Tyr Thr Leu Ser Leu Ser Gly Ala Gly Tyr
           435                 440                 445
   aca gcc ggc cag caa ttg acg gag gtc att ggc tgc acg acc gtg acg      1392
   Thr Ala Gly Gln Gln Leu Thr Glu Val Ile Gly Cys Thr Thr Val Thr
       450                 455                 460
   gtt ggt tcg gat gga aat gtg cct gtt cct atg gca ggt ggg cta cct      1440
   Val Gly Ser Asp Gly Asn Val Pro Val Pro Met Ala Gly Gly Leu Pro
   465                 470                 475                 480
   agg gta ttg tat ccg act gag aag ttg gca ggt agc aag atc tgt agt      1488
   Arg Val Leu Tyr Pro Thr Glu Lys Leu Ala Gly Ser Lys Ile Cys Ser
                   485                 490                 495
   agc tcg gga aga aca acc acg acc aca act gct gct gct act agt aca      1536
   Ser Ser Gly Arg Thr Thr Thr Thr Thr Thr Ala Ala Ala Thr Ser Thr
               500                 505                 510
   tcc aaa gcc acc acc tcc tct tct tct tct tct gct gct gct act act      1584
   Ser Lys Ala Thr Thr Ser Ser Ser Ser Ser Ser Ala Ala Ala Thr Thr
           515                 520                 525
   tct tca tca tgc acc gca aca agc acc acc ctc ccc atc acc ttc gaa      1632
   Ser Ser Ser Cys Thr Ala Thr Ser Thr Thr Leu Pro Ile Thr Phe Glu
       530                 535                 540
   gaa ctc gtc acc act acc tac ggg gaa gaa gtc tac ctc agc gga tct      1680
   Glu Leu Val Thr Thr Thr Tyr Gly Glu Glu Val Tyr Leu Ser Gly Ser
   545                 550                 555                 560
   atc tcc cag ctc gga gag tgg gat acg agt gac gcg gtg aag ttg tcc      1728
   Ile Ser Gln Leu Gly Glu Trp Asp Thr Ser Asp Ala Val Lys Leu Ser
                   565                 570                 575
   gcg gat gat tat acc tcg agt aac ccc gag tgg tct gtt act gtg tcg      1776
   Ala Asp Asp Tyr Thr Ser Ser Asn Pro Glu Trp Ser Val Thr Val Ser
               580                 585                 590
   ttg ccg gtg ggg acg acc ttc gag tat aag ttt att aag gtc gat gag      1824
   Leu Pro Val Gly Thr Thr Phe Glu Tyr Lys Phe Ile Lys Val Asp Glu
           595                 600                 605
   ggt gga agt gtg act tgg gaa agt gat ccg aat agg gag tat act gtg      1872
   Gly Gly Ser Val Thr Trp Glu Ser Asp Pro Asn Arg Glu Tyr Thr Val
       610                 615                 620
   cct gaa tgt ggg aat ggg agt ggg gag acg gtg gtt gat acg tgg agg      1920
   Pro Glu Cys Gly Asn Gly Ser Gly Glu Thr Val Val Asp Thr Trp Arg
   625                 630                 635                 640
   tag                                                                  1923
<210> 36
   <211> 640
   <212> PRT
   <213> Artificial
<220>
   <223> Artificial
<400> 36
   Met Val Ala Trp Trp Ser Leu Phe Leu Tyr Gly Leu Gln Val Ala Ala
   1               5                   10                  15
   Pro Ala Leu Ala Ala Thr Pro Ala Asp Trp Arg Ser Gln Ser Ile Tyr
               20                  25                  30
   Phe Leu Leu Thr Asp Arg Phe Ala Arg Thr Asp Gly Ser Thr Thr Ala
           35                  40                  45
   Thr Cys Asn Thr Ala Asp Gln Lys Tyr Cys Gly Gly Thr Trp Gln Gly
       50                  55                  60
   Ile Ile Asp Lys Leu Asp Tyr Ile Gln Gly Met Gly Phe Thr Ala Ile
   65                  70                  75                  80
   Trp Ile Thr Pro Val Thr Ala Gln Leu Pro Gln Thr Thr Ala Tyr Gly
                   85                  90                  95
   Asp Ala Tyr His Gly Tyr Trp Gln Gln Asp Ile Tyr Ser Leu Asn Glu
               100                 105                 110
   Asn Tyr Gly Thr Ala Asp Asp Leu Lys Ala Leu Ser Ser Ala Leu His
           115                 120                 125
   Glu Arg Gly Met Tyr Leu Met Val Asp Val Val Ala Asn His Met Gly
       130                 135                 140
   Tyr Asp Gly Ala Gly Ser Ser Val Asp Tyr Ser Val Phe Lys Pro Phe
   145                 150                 155                 160
   Ser Ser Gln Asp Tyr Phe His Pro Phe Cys Phe Ile Gln Asn Tyr Glu
                   165                 170                 175
   Asp Gln Thr Gln Val Glu Asp Cys Trp Leu Gly Asp Asn Thr Val Ser
               180                 185                 190
   Leu Pro Asp Leu Asp Thr Thr Lys Asp Val Val Lys Asn Glu Trp Tyr
           195                 200                 205
   Asp Trp Val Gly Ser Leu Val Ser Asn Tyr Ser Ile Asp Gly Leu Arg
       210                 215                 220
   Ile Asp Thr Val Lys His Val Gln Lys Asp Phe Trp Pro Gly Tyr Asn
   225                 230                 235                 240
   Lys Ala Ala Gly Val Tyr Cys Ile Gly Glu Val Leu Asp Gly Asp Pro
   245 250 255
   Ala Tyr Thr Cys Pro Tyr Gln Asn Val Met Asp Gly Val Leu Asn Tyr
   260 265 270
   Pro Ile Tyr Tyr Pro Leu Leu Asn Ala Phe Lys Ser Thr Ser Gly Ser
   275 280 285
   Met Asp Asp Leu Tyr Asn Met Ile Asn Thr Val Lys Ser Asp Cys Pro
   290 295 300
   Asp Ser Thr Leu Leu Gly Thr Phe Val Glu Asn His Asp Asn Pro Arg
   305 310 315 320
   Phe Ala Ser Tyr Thr Asn Asp Ile Ala Leu Ala Lys Asn Val Ala Ala
   325 330 335
   Phe Ile Ile Leu Asn Asp Gly Ile Pro Ile Ile Tyr Ala Gly Gln Glu
   340 345 350
   Gln His Tyr Ala Gly Gly Asn Asp Pro Ala Asn Arg Glu Ala Thr Trp
   355 360 365
   Leu Ser Gly Tyr Pro Thr Asp Ser Glu Leu Tyr Lys Leu Ile Ala Ser
   370 375 380
   Ala Asn Ala Ile Arg Asn Tyr Ala Ile Ser Lys Asp Thr Gly Phe Val
   385 390 395 400
   Thr Tyr Lys Asn Trp Pro Ile Tyr Lys Asp Asp Thr Thr Ile Ala Met
   405 410 415
   Arg Lys Gly Thr Asp Gly Ser Gln Ile Val Thr Ile Leu Ser Asn Lys
   420 425 430
   Gly Ala Ser Gly Asp Ser Tyr Thr Leu Ser Leu Ser Gly Ala Gly Tyr
   435 440 445
   Thr Ala Gly Gln Gln Leu Thr Glu Val Ile Gly Cys Thr Thr Val Thr
   450 455 460
   Val Gly Ser Asp Gly Asn Val Pro Val Pro Met Ala Gly Gly Leu Pro
   465 470 475 480
   Arg Val Leu Tyr Pro Thr Glu Lys Leu Ala Gly Ser Lys Ile Cys Ser
   485 490 495
   Ser Ser Gly Arg Thr Thr Thr Thr Thr Thr Ala Ala Ala Thr Ser Thr
               500                 505                 510
   Ser Lys Ala Thr Thr Ser Ser Ser Ser Ser Ser Ala Ala Ala Thr Thr
           515                 520                 525
   Ser Ser Ser Cys Thr Ala Thr Ser Thr Thr Leu Pro Ile Thr Phe Glu
       530                 535                 540
   Glu Leu Val Thr Thr Thr Tyr Gly Glu Glu Val Tyr Leu Ser Gly Ser
   545                 550                 555                 560
   Ile Ser Gln Leu Gly Glu Trp Asp Thr Ser Asp Ala Val Lys Leu Ser
                   565                 570                 575
   Ala Asp Asp Tyr Thr Ser Ser Asn Pro Glu Trp Ser Val Thr Val Ser
               580                 585                 590
   Leu Pro Val Gly Thr Thr Phe Glu Tyr Lys Phe Ile Lys Val Asp Glu
           595                 600                 605
   Gly Gly Ser Val Thr Trp Glu Ser Asp Pro Asn Arg Glu Tyr Thr Val
       610                 615                 620
   Pro Glu Cys Gly Asn Gly Ser Gly Glu Thr Val Val Asp Thr Trp Arg
   625                 630                 635                 640
<210> 37
   <211> 1830
   <212> DNA
   <213> Artificial
<220>
   <223> Artificial
<220>
   <221> CDS
   <222> (1)..(1830)
<400> 37
   atg aga tta tcg act tcg agt ctc ttc ctt tcc gtg tct ctg ctg ggg      48
   Met Arg Leu Ser Thr Ser Ser Leu Phe Leu Ser Val Ser Leu Leu Gly
   1               5                   10                  15
   aag ctg gcc ctc ggg ctg tcg gct gca gaa tgg cgc act cag tcg att      96
   Lys Leu Ala Leu Gly Leu Ser Ala Ala Glu Trp Arg Thr Gln Ser Ile
               20                  25                  30
   tac ttc cta ttg acg gat cgg ttc ggt agg acg gac aat tcg acg aca     144
   Tyr Phe Leu Leu Thr Asp Arg Phe Gly Arg Thr Asp Asn Ser Thr Thr
           35                  40                  45
   gct aca tgc gat acg ggt gac caa atc tat tgt ggt ggc agt tgg caa      192
   Ala Thr Cys Asp Thr Gly Asp Gln Ile Tyr Cys Gly Gly Ser Trp Gln
       50                  55                  60
   gga atc atc aac cat ctg gat tat atc cag ggc atg gga ttc acg gcc      240
   Gly Ile Ile Asn His Leu Asp Tyr Ile Gln Gly Met Gly Phe Thr Ala
   65                  70                  75                  80
   atc tgg atc tcg cct atc act gaa cag ctg ccc cag gat act gct gat      288
   Ile Trp Ile Ser Pro Ile Thr Glu Gln Leu Pro Gln Asp Thr Ala Asp
                   85                  90                  95
   ggt gaa gct tac cat gga tat tgg cag cag aag ata tac gac gtg aac      336
   Gly Glu Ala Tyr His Gly Tyr Trp Gln Gln Lys Ile Tyr Asp Val Asn
               100                 105                 110
   tcc aac ttc ggc act gca gat gac ctc aag tcc ctc tca gat gcg ctt      384
   Ser Asn Phe Gly Thr Ala Asp Asp Leu Lys Ser Leu Ser Asp Ala Leu
           115                 120                 125
   cat gcc cgc gga atg tac ctc atg gtg gac gtc gtc cct aac cac atg      432
   His Ala Arg Gly Met Tyr Leu Met Val Asp Val Val Pro Asn His Met
       130                 135                 140
   ggc tac gcc ggc aac ggc aac gat gta gac tac agc gtc ttc gac ccc      480
   Gly Tyr Ala Gly Asn Gly Asn Asp Val Asp Tyr Ser Val Phe Asp Pro
   145                 150                 155                 160
   ttc gat tcc tcc tcc tac ttc cac cca tac tgc ctg atc aca gat tgg      528
   Phe Asp Ser Ser Ser Tyr Phe His Pro Tyr Cys Leu Ile Thr Asp Trp
                   165                 170                 175
   gac aac ttg acc atg gtc caa gat tgt tgg gag ggt gac acc atc gta      576
   Asp Asn Leu Thr Met Val Gln Asp Cys Trp Glu Gly Asp Thr Ile Val
               180                 185                 190
   tct ctg cca gac cta aac acc acc gaa act gcc gtg aga aca atc tgg      624
   Ser Leu Pro Asp Leu Asn Thr Thr Glu Thr Ala Val Arg Thr Ile Trp
           195                 200                 205
   tat gac tgg gta gcc gac ctg gta tcc aat tat tca gtc gac gga ctc      672
   Tyr Asp Trp Val Ala Asp Leu Val Ser Asn Tyr Ser Val Asp Gly Leu
       210                 215                 220
   cgc atc gac agt gtc ctc gaa gtc gaa cca gac ttc ttc ccg ggc tac      720
   Arg Ile Asp Ser Val Leu Glu Val Glu Pro Asp Phe Phe Pro Gly Tyr
   225                 230                 235                 240
   cag gaa gca gca ggt gtc tac tgc gtc ggc gaa gtc gac aac ggc aac      768
   Gln Glu Ala Ala Gly Val Tyr Cys Val Gly Glu Val Asp Asn Gly Asn
                   245                 250                 255
   cct gcc ctc gac tgc cca tac cag aag gtc ctg gac ggc gtc ctc aac      816
   Pro Ala Leu Asp Cys Pro Tyr Gln Lys Val Leu Asp Gly Val Leu Asn
               260                 265                 270
   tat ccg atc tac tgg caa ctc ctc tac gcc ttc gaa tcc tcc agc ggc      864
   Tyr Pro Ile Tyr Trp Gln Leu Leu Tyr Ala Phe Glu Ser Ser Ser Gly
           275                     280                     285
   agc atc agc aat ctc tac aac atg atc aaa tcc gtc gca agc gac tgc        912
   Ser Ile Ser Asn Leu Tyr Asn Met Ile Lys Ser Val Ala Ser Asp Cys
       290                     295                     300
   tcc gat ccg aca cta ctc ggc aac ttc atc gaa aac cac gac aat ccc        960
   Ser Asp Pro Thr Leu Leu Gly Asn Phe Ile Glu Asn His Asp Asn Pro
   305                     310                     315                     320
   cgt ttc gcc tcc tac acc tcc gac tac tcg caa gcc aaa aac gtc ctc       1008
   Arg Phe Ala Ser Tyr Thr Ser Asp Tyr Ser Gln Ala Lys Asn Val Leu
                   325                     330                     335
   agc tac atc ttc ctc tcc gac ggc atc ccc atc gtc tac gcc ggc gaa       1056
   Ser Tyr Ile Phe Leu Ser Asp Gly Ile Pro Ile Val Tyr Ala Gly Glu
               340                     345                     350
   gaa cag cac tac tcc ggc ggc aag gtg ccc tac aac cgc gaa gcg acc       1104
   Glu Gln His Tyr Ser Gly Gly Lys Val Pro Tyr Asn Arg Glu Ala Thr
           355                     360                     365
   tgg ctt tca ggc tac gac acc tcc gca gag ctg tac acc tgg ata gcc       1152
   Trp Leu Ser Gly Tyr Asp Thr Ser Ala Glu Leu Tyr Thr Trp Ile Ala
       370                     375                     380
   acc acg aac gcg atc cgc aaa cta gcc atc tca gct gac tcg gcc tac       1200
   Thr Thr Asn Ala Ile Arg Lys Leu Ala Ile Ser Ala Asp Ser Ala Tyr
   385                     390                     395                     400
   att acc tac gcg aat gat gca ttc tac act gac agc aac acc atc gca       1248
   Ile Thr Tyr Ala Asn Asp Ala Phe Tyr Thr Asp Ser Asn Thr Ile Ala
                   405                     410                     415
   atg cgc aaa ggc acc tca ggg agc caa gtc atc acc gtc ctc tcc aac       1296
   Met Arg Lys Gly Thr Ser Gly Ser Gln Val Ile Thr Val Leu Ser Asn
               420                     425                     430
   aaa ggc tcc tca gga agc agc tac acc ctg acc ctc agc gga agc ggc       1344
   Lys Gly Ser Ser Gly Ser Ser Tyr Thr Leu Thr Leu Ser Gly Ser Gly
           435                     440                     445
   tac aca tcc ggc acg aag ctg atc gaa gcg tac aca tgc aca tcc gtg       1392
   Tyr Thr Ser Gly Thr Lys Leu Ile Glu Ala Tyr Thr Cys Thr Ser Val
       450                     455                     460
   acc gtg gac tcg agc ggc gat att ccc gtg ccg atg gcg tcg gga tta       1440
   Thr Val Asp Ser Ser Gly Asp Ile Pro Val Pro Met Ala Ser Gly Leu
   465                     470                     475                     480
   ccg aga gtt ctt ctg ccc gcg tcc gtc gtc gat agc tct tcg ctc tgt       1488
   Pro Arg Val Leu Leu Pro Ala Ser Val Val Asp Ser Ser Ser Leu Cys
                   485                     490                     495
   ggc ggg agc gga aga ggt gct aca agc ccg ggt ggc tcc tcg ggt agt       1536
   Gly Gly Ser Gly Arg Gly Ala Thr Ser Pro Gly Gly Ser Ser Gly Ser
               500                     505                     510
   gtc gag gtc act ttc gac gtt tac gct acc aca gta tat ggc cag aac       1584
   Val Glu Val Thr Phe Asp Val Tyr Ala Thr Thr Val Tyr Gly Gln Asn
           515                     520                     525
   atc tat atc acc ggt gat gtg agt gag ctc ggc aac tgg aca ccc gcc      1632
   Ile Tyr Ile Thr Gly Asp Val Ser Glu Leu Gly Asn Trp Thr Pro Ala
       530                 535                 540
   aat ggt gtt gca ctc tct tct gct aac tac ccc acc tgg agt gcc acg      1680
   Asn Gly Val Ala Leu Ser Ser Ala Asn Tyr Pro Thr Trp Ser Ala Thr
   545                 550                 555                 560
   atc gct ctc ccc gct gac acg aca atc cag tac aag tat gtc aac att      1728
   Ile Ala Leu Pro Ala Asp Thr Thr Ile Gln Tyr Lys Tyr Val Asn Ile
                   565                 570                 575
   gac ggc agc acc gtc atc tgg gag gat gct atc agc aat cgc gag atc      1776
   Asp Gly Ser Thr Val Ile Trp Glu Asp Ala Ile Ser Asn Arg Glu Ile
               580                 585                 590
   acg acg ccc gcc agc ggc aca tac acc gaa aaa gac act tgg gat gaa      1824
   Thr Thr Pro Ala Ser Gly Thr Tyr Thr Glu Lys Asp Thr Trp Asp Glu
           595                 600                 605
   tct tag                                                              1830
   Ser
<210> 38
   <211> 609
   <212> PRT
   <213> Artificial
<220>
   <223> Artificial
<400> 38
   Met Arg Leu Ser Thr Ser Ser Leu Phe Leu Ser Val Ser Leu Leu Gly
   1               5                   10                  15
   Lys Leu Ala Leu Gly Leu Ser Ala Ala Glu Trp Arg Thr Gln Ser Ile
               20                  25                  30
   Tyr Phe Leu Leu Thr Asp Arg Phe Gly Arg Thr Asp Asn Ser Thr Thr
           35                  40                  45
   Ala Thr Cys Asp Thr Gly Asp Gln Ile Tyr Cys Gly Gly Ser Trp Gln
       50                  55                  60
   Gly Ile Ile Asn His Leu Asp Tyr Ile Gln Gly Met Gly Phe Thr Ala
   65                  70                  75                  80
   Ile Trp Ile Ser Pro Ile Thr Glu Gln Leu Pro Gln Asp Thr Ala Asp
                   85                  90                  95
   Gly Glu Ala Tyr His Gly Tyr Trp Gln Gln Lys Ile Tyr Asp Val Asn
   100 105 110
   Ser Asn Phe Gly Thr Ala Asp Asp Leu Lys Ser Leu Ser Asp Ala Leu
   115 120 125
   His Ala Arg Gly Met Tyr Leu Met Val Asp Val Val Pro Asn His Met
   130 135 140
   Gly Tyr Ala Gly Asn Gly Asn Asp Val Asp Tyr Ser Val Phe Asp Pro
   145 150 155 160
   Phe Asp Ser Ser Ser Tyr Phe His Pro Tyr Cys Leu Ile Thr Asp Trp
   165 170 175
   Asp Asn Leu Thr Met Val Gln Asp Cys Trp Glu Gly Asp Thr Ile Val
   180 185 190
   Ser Leu Pro Asp Leu Asn Thr Thr Glu Thr Ala Val Arg Thr Ile Trp
   195 200 205
   Tyr Asp Trp Val Ala Asp Leu Val Ser Asn Tyr Ser Val Asp Gly Leu
   210 215 220
   Arg Ile Asp Ser Val Leu Glu Val Glu Pro Asp Phe Phe Pro Gly Tyr
   225 230 235 240
   Gln Glu Ala Ala Gly Val Tyr Cys Val Gly Glu Val Asp Asn Gly Asn
   245 250 255
   Pro Ala Leu Asp Cys Pro Tyr Gln Lys Val Leu Asp Gly Val Leu Asn
   260 265 270
   Tyr Pro Ile Tyr Trp Gln Leu Leu Tyr Ala Phe Glu Ser Ser Ser Gly
   275 280 285
   Ser Ile Ser Asn Leu Tyr Asn Met Ile Lys Ser Val Ala Ser Asp Cys
   290 295 300
   Ser Asp Pro Thr Leu Leu Gly Asn Phe Ile Glu Asn His Asp Asn Pro
   305 310 315 320
   Arg Phe Ala Ser Tyr Thr Ser Asp Tyr Ser Gln Ala Lys Asn Val Leu
   325 330 335
   Ser Tyr Ile Phe Leu Ser Asp Gly Ile Pro Ile Val Tyr Ala Gly Glu
   340 345 350
   Glu Gln His Tyr Ser Gly Gly Lys Val Pro Tyr Asn Arg Glu Ala Thr
   355 360 365
   Trp Leu Ser Gly Tyr Asp Thr Ser Ala Glu Leu Tyr Thr Trp Ile Ala
   370 375 380
   Thr Thr Asn Ala Ile Arg Lys Leu Ala Ile Ser Ala Asp Ser Ala Tyr
   385 390 395 400
   Ile Thr Tyr Ala Asn Asp Ala Phe Tyr Thr Asp Ser Asn Thr Ile Ala
   405 410 415
   Met Arg Lys Gly Thr Ser Gly Ser Gln Val Ile Thr Val Leu Ser Asn
   420 425 430
   Lys Gly Ser Ser Gly Ser Ser Tyr Thr Leu Thr Leu Ser Gly Ser Gly
   435 440 445
   Tyr Thr Ser Gly Thr Lys Leu Ile Glu Ala Tyr Thr Cys Thr Ser Val
   450 455 460
   Thr Val Asp Ser Ser Gly Asp Ile Pro Val Pro Met Ala Ser Gly Leu
   465 470 475 480
   Pro Arg Val Leu Leu Pro Ala Ser Val Val Asp Ser Ser Ser Leu Cys
   485 490 495
   Gly Gly Ser Gly Arg Gly Ala Thr Ser Pro Gly Gly Ser Ser Gly Ser
   500 505 510
   Val Glu Val Thr Phe Asp Val Tyr Ala Thr Thr Val Tyr Gly Gln Asn
   515 520 525
   Ile Tyr Ile Thr Gly Asp Val Ser Glu Leu Gly Asn Trp Thr Pro Ala
   530 535 540
   Asn Gly Val Ala Leu Ser Ser Ala Asn Tyr Pro Thr Trp Ser Ala Thr
   545 550 555 560
   Ile Ala Leu Pro Ala Asp Thr Thr Ile Gln Tyr Lys Tyr Val Asn Ile
   565 570 575
   Asp Gly Ser Thr Val Ile Trp Glu Asp Ala Ile Ser Asn Arg Glu Ile
   580 585 590
   Thr Thr Pro Ala Ser Gly Thr Tyr Thr Glu Lys Asp Thr Trp Asp Glu
           595                 600                 605
   Ser
<210> 39
   <211> 1827
   <212> DNA
   <213> Artificial
<220>
   <223> Artificial
<220>
   <221> CDS
   <222> (1)..(1827)
<400> 39
   atg gtc gcg tgg tgg tct cta ttt ctg tac ggc ctt cag gtc gcg gca      48
   Met Val Ala Trp Trp Ser Leu Phe Leu Tyr Gly Leu Gln Val Ala Ala
   1               5                   10                  15
   cct gct ttg gct gca acg cct gcg gac tgg cga tcg caa tcc att tat      96
   Pro Ala Leu Ala Ala Thr Pro Ala Asp Trp Arg Ser Gln Ser Ile Tyr
               20                  25                  30
   ttc ctt ctc acg gat cga ttt gca agg acg gat ggg tcg acg act gcg     144
   Phe Leu Leu Thr Asp Arg Phe Ala Arg Thr Asp Gly Ser Thr Thr Ala
           35                  40                  45
   act tgt aat act gcg gat cag aaa tac tgt ggt gga aca tgg cag ggc     192
   Thr Cys Asn Thr Ala Asp Gln Lys Tyr Cys Gly Gly Thr Trp Gln Gly
       50                  55                  60
   atc atc gac aag ttg gac tat atc cag gga atg ggc ttc aca gcc atc     240
   Ile Ile Asp Lys Leu Asp Tyr Ile Gln Gly Met Gly Phe Thr Ala Ile
   65                  70                  75                  80
   tgg atc acc ccc gtt aca gcc cag ctg ccc cag acc acc gca tat gga     288
   Trp Ile Thr Pro Val Thr Ala Gln Leu Pro Gln Thr Thr Ala Tyr Gly
                   85                  90                  95
   gat gcc tac cat ggc tac tgg cag cag gat ata tac tct ctg aac gaa     336
   Asp Ala Tyr His Gly Tyr Trp Gln Gln Asp Ile Tyr Ser Leu Asn Glu
               100                 105                 110
   aac tac ggc act gca gat gac ttg aag gcg ctc tct tcg gcc ctt cat     384
   Asn Tyr Gly Thr Ala Asp Asp Leu Lys Ala Leu Ser Ser Ala Leu His
           115                 120                 125
   gag agg ggg atg tat ctt atg gtc gat gtg gtt gct aac cat atg ggc     432
   Glu Arg Gly Met Tyr Leu Met Val Asp Val Val Ala Asn His Met Gly
       130                 135                 140
   tat gat gga gcg ggt agc tca gtc gat tac agt gtg ttt aaa ccg ttc     480
   Tyr Asp Gly Ala Gly Ser Ser Val Asp Tyr Ser Val Phe Lys Pro Phe
   145                 150                 155                 160
   agt tcc caa gac tac ttc cac ccg ttc tgt ttc att caa aac tat gaa      528
   Ser Ser Gln Asp Tyr Phe His Pro Phe Cys Phe Ile Gln Asn Tyr Glu
                   165                 170                 175
   gat cag act cag gtt gag gat tgc tgg cta gga gat aac act gtc tcc      576
   Asp Gln Thr Gln Val Glu Asp Cys Trp Leu Gly Asp Asn Thr Val Ser
               180                 185                 190
   ttg cct gat ctc gat acc acc aag gat gtg gtc aag aat gaa tgg tac      624
   Leu Pro Asp Leu Asp Thr Thr Lys Asp Val Val Lys Asn Glu Trp Tyr
           195                 200                 205
   gac tgg gtg gga tca ttg gta tcg aac tac tcc att gac ggc ctc cgt      672
   Asp Trp Val Gly Ser Leu Val Ser Asn Tyr Ser Ile Asp Gly Leu Arg
       210                 215                 220
   atc gac aca gta aaa cac gtc cag aag gac ttc tgg ccc ggg tac aac      720
   Ile Asp Thr Val Lys His Val Gln Lys Asp Phe Trp Pro Gly Tyr Asn
   225                 230                 235                 240
   aaa gcc gca ggc gtg tac tgt atc ggc gag gtg ctc gac ggt gat ccg      768
   Lys Ala Ala Gly Val Tyr Cys Ile Gly Glu Val Leu Asp Gly Asp Pro
                   245                 250                 255
   gcc tac act tgt ccc tac cag aac gtc atg gac ggc gta ctg aac tat      816
   Ala Tyr Thr Cys Pro Tyr Gln Asn Val Met Asp Gly Val Leu Asn Tyr
               260                 265                 270
   ccc att tac tat cca ctc ctc aac gcc ttc aag tca acc tcc ggc agc      864
   Pro Ile Tyr Tyr Pro Leu Leu Asn Ala Phe Lys Ser Thr Ser Gly Ser
           275                 280                 285
   atg gac gac ctc tac aac atg atc aac acc gtc aaa tcc gac tgt cca      912
   Met Asp Asp Leu Tyr Asn Met Ile Asn Thr Val Lys Ser Asp Cys Pro
       290                 295                 300
   gac tca aca ctc ctg ggc aca ttc gtc gag aac cac gac aac cca cgg      960
   Asp Ser Thr Leu Leu Gly Thr Phe Val Glu Asn His Asp Asn Pro Arg
   305                 310                 315                 320
   ttc gct tct tac acc aac gac ata gcc ctc gcc aag aac gtc gca gca     1008
   Phe Ala Ser Tyr Thr Asn Asp Ile Ala Leu Ala Lys Asn Val Ala Ala
                   325                 330                 335
   ttc atc atc ctc aac gac gga atc ccc atc atc tac gcc ggc caa gaa     1056
   Phe Ile Ile Leu Asn Asp Gly Ile Pro Ile Ile Tyr Ala Gly Gln Glu
               340                 345                 350
   cag cac tac gcc ggc gga aac gac ccc gcg aac cgc gaa gca acc tgg     1104
   Gln His Tyr Ala Gly Gly Asn Asp Pro Ala Asn Arg Glu Ala Thr Trp
           355                 360                 365
   ctc tcg ggc tac ccg acc gac agc gag ctg tac aag tta att gcc tcc     1152
   Leu Ser Gly Tyr Pro Thr Asp Ser Glu Leu Tyr Lys Leu Ile Ala Ser
       370                 375                 380
   gcg aac gca atc cgg aac tat gcc att agc aaa gat aca gga ttc gtg     1200
   Ala Asn Ala Ile Arg Asn Tyr Ala Ile Ser Lys Asp Thr Gly Phe Val
   385                 390                 395                 400
   acc tac aag aac tgg ccc atc tac aaa gac gac aca acg atc gcc atg      1248
   Thr Tyr Lys Asn Trp Pro Ile Tyr Lys Asp Asp Thr Thr Ile Ala Met
                   405                 410                 415
   cgc aag ggc aca gat ggg tcg cag atc gtg act atc ttg tcc aac aag      1296
   Arg Lys Gly Thr Asp Gly Ser Gln Ile Val Thr Ile Leu Ser Asn Lys
               420                 425                 430
   ggt gct tcg ggt gat tcg tat acc ctc tcc ttg agt ggt gcg ggt tac      1344
   Gly Ala Ser Gly Asp Ser Tyr Thr Leu Ser Leu Ser Gly Ala Gly Tyr
           435                 440                 445
   aca gcc ggc cag caa ttg acg gag gtc att ggc tgc acg acc gtg acg      1392
   Thr Ala Gly Gln Gln Leu Thr Glu Val Ile Gly Cys Thr Thr Val Thr
       450                 455                 460
   gtt ggt tcg gat gga aat gtg cct gtt cct atg gca ggt ggg cta cct      1440
   Val Gly Ser Asp Gly Asn Val Pro Val Pro Met Ala Gly Gly Leu Pro
   465                 470                 475                 480
   agg gta ttg tat ccg act gag aag ttg gca ggt agc aag atc tgt agt      1488
   Arg Val Leu Tyr Pro Thr Glu Lys Leu Ala Gly Ser Lys Ile Cys Ser
                   485                 490                 495
   agc tcg gga aga ggt gct aca agc ccg ggt ggc tcc tcg ggt agt gtc      1536
   Ser Ser Gly Arg Gly Ala Thr Ser Pro Gly Gly Ser Ser Gly Ser Val
               500                 505                 510
   gag gtc act ttc gac gtt tac gct acc aca gta tat ggc cag aac atc      1584
   Glu Val Thr Phe Asp Val Tyr Ala Thr Thr Val Tyr Gly Gln Asn Ile
           515                 520                 525
   tat atc acc ggt gat gtg agt gag ctc ggc aac tgg aca ccc gcc aat      1632
   Tyr Ile Thr Gly Asp Val Ser Glu Leu Gly Asn Trp Thr Pro Ala Asn
       530                 535                 540
   ggt gtt gca ctc tct tct gct aac tac ccc acc tgg agt gcc acg atc      1680
   Gly Val Ala Leu Ser Ser Ala Asn Tyr Pro Thr Trp Ser Ala Thr Ile
   545                 550                 555                 560
   gct ctc ccc gct gac acg aca atc cag tac aag tat gtc aac att gac      1728
   Ala Leu Pro Ala Asp Thr Thr Ile Gln Tyr Lys Tyr Val Asn Ile Asp
                   565                 570                 575
   ggc agc acc gtc atc tgg gag gat gct atc agc aat cgc gag atc acg      1776
   Gly Ser Thr Val Ile Trp Glu Asp Ala Ile Ser Asn Arg Glu Ile Thr
               580                 585                 590
   acg ccc gcc agc ggc aca tac acc gaa aaa gac act tgg gat gaa tct      1824
   Thr Pro Ala Ser Gly Thr Tyr Thr Glu Lys Asp Thr Trp Asp Glu Ser
           595                 600                 605
   tag                                                                  1827
<210> 40
   <211> 608
   <212> PRT
   <213> Artificial
<220>
   <223> Artificial
<400> 40
   Met Val Ala Trp Trp Ser Leu Phe Leu Tyr Gly Leu Gln Val Ala Ala
   1               5                   10                  15
   Pro Ala Leu Ala Ala Thr Pro Ala Asp Trp Arg Ser Gln Ser Ile Tyr
               20                  25                  30
   Phe Leu Leu Thr Asp Arg Phe Ala Arg Thr Asp Gly Ser Thr Thr Ala
           35                  40                  45
   Thr Cys Asn Thr Ala Asp Gln Lys Tyr Cys Gly Gly Thr Trp Gln Gly
       50                  55                  60
   Ile Ile Asp Lys Leu Asp Tyr Ile Gln Gly Met Gly Phe Thr Ala Ile
   65                  70                  75                  80
   Trp Ile Thr Pro Val Thr Ala Gln Leu Pro Gln Thr Thr Ala Tyr Gly
                   85                  90                  95
   Asp Ala Tyr His Gly Tyr Trp Gln Gln Asp Ile Tyr Ser Leu Asn Glu
               100                 105                 110
   Asn Tyr Gly Thr Ala Asp Asp Leu Lys Ala Leu Ser Ser Ala Leu His
           115                 120                 125
   Glu Arg Gly Met Tyr Leu Met Val Asp Val Val Ala Asn His Met Gly
       130                 135                 140
   Tyr Asp Gly Ala Gly Ser Ser Val Asp Tyr Ser Val Phe Lys Pro Phe
   145                 150                 155                 160
   Ser Ser Gln Asp Tyr Phe His Pro Phe Cys Phe Ile Gln Asn Tyr Glu
                   165                 170                 175
   Asp Gln Thr Gln Val Glu Asp Cys Trp Leu Gly Asp Asn Thr Val Ser
               180                 185                 190
   Leu Pro Asp Leu Asp Thr Thr Lys Asp Val Val Lys Asn Glu Trp Tyr
           195                 200                 205
   Asp Trp Val Gly Ser Leu Val Ser Asn Tyr Ser Ile Asp Gly Leu Arg
       210                 215                 220
   Ile Asp Thr Val Lys His Val Gln Lys Asp Phe Trp Pro Gly Tyr Asn
   225                 230                 235                 240
   Lys Ala Ala Gly Val Tyr Cys Ile Gly Glu Val Leu Asp Gly Asp Pro
                   245                 250                 255
   Ala Tyr Thr Cys Pro Tyr Gln Asn Val Met Asp Gly Val Leu Asn Tyr
               260                 265                 270
   Pro Ile Tyr Tyr Pro Leu Leu Asn Ala Phe Lys Ser Thr Ser Gly Ser
           275                 280                 285
   Met Asp Asp Leu Tyr Asn Met Ile Asn Thr Val Lys Ser Asp Cys Pro
       290                 295                 300
   Asp Ser Thr Leu Leu Gly Thr Phe Val Glu Asn His Asp Asn Pro Arg
   305                 310                 315                 320
   Phe Ala Ser Tyr Thr Asn Asp Ile Ala Leu Ala Lys Asn Val Ala Ala
                   325                 330                 335
   Phe Ile Ile Leu Asn Asp Gly Ile Pro Ile Ile Tyr Ala Gly Gln Glu
               340                 345                 350
   Gln His Tyr Ala Gly Gly Asn Asp Pro Ala Asn Arg Glu Ala Thr Trp
           355                 360                 365
   Leu Ser Gly Tyr Pro Thr Asp Ser Glu Leu Tyr Lys Leu Ile Ala Ser
       370                 375                 380
   Ala Asn Ala Ile Arg Asn Tyr Ala Ile Ser Lys Asp Thr Gly Phe Val
   385                 390                 395                 400
   Thr Tyr Lys Asn Trp Pro Ile Tyr Lys Asp Asp Thr Thr Ile Ala Met
                   405                 410                 415
   Arg Lys Gly Thr Asp Gly Ser Gln Ile Val Thr Ile Leu Ser Asn Lys
               420                 425                 430
   Gly Ala Ser Gly Asp Ser Tyr Thr Leu Ser Leu Ser Gly Ala Gly Tyr
           435                 440                 445
   Thr Ala Gly Gln Gln Leu Thr Glu Val Ile Gly Cys Thr Thr Val Thr
       450                 455                 460
   Val Gly Ser Asp Gly Asn Val Pro Val Pro Met Ala Gly Gly Leu Pro
   465 470 475 480
   Arg Val Leu Tyr Pro Thr Glu Lys Leu Ala Gly Ser Lys Ile Cys Ser
   485 490 495
   Ser Ser Gly Arg Gly Ala Thr Ser Pro Gly Gly Ser Ser Gly Ser Val
   500 505 510
   Glu Val Thr Phe Asp Val Tyr Ala Thr Thr Val Tyr Gly Gln Asn Ile
   515 520 525
   Tyr Ile Thr Gly Asp Val Ser Glu Leu Gly Asn Trp Thr Pro Ala Asn
   530 535 540
   Gly Val Ala Leu Ser Ser Ala Asn Tyr Pro Thr Trp Ser Ala Thr Ile
   545 550 555 560
   Ala Leu Pro Ala Asp Thr Thr Ile Gln Tyr Lys Tyr Val Asn Ile Asp
   565 570 575
   Gly Ser Thr Val Ile Trp Glu Asp Ala Ile Ser Asn Arg Glu Ile Thr
   580 585 590
   Thr Pro Ala Ser Gly Thr Tyr Thr Glu Lys Asp Thr Trp Asp Glu Ser
   595 600 605
<210> 41
   <211> 640
   <212> PRT
   <213> Aspergillus kawachii
<220>
   <221> mat_peptide
   <222> (22)..(640)
<400> 41
   Met Arg Val Ser Thr Ser Ser Ile Ala Leu Ala Val Ser Leu Phe Gly
   -20 -15 -10
   Lys Leu Ala Leu Gly Leu Ser Ala Ala Glu Trp Arg Thr Gln Ser Ile
   -5 -1 1 5 10
   Tyr Phe Leu Leu Thr Asp Arg Phe Gly Arg Thr Asp Asn Ser Thr Thr
   15 20 25
   Ala Thr Cys Asn Thr Gly Asp Gln Ile Tyr Cys Gly Gly Ser Trp Gln
   30 35 40
   Gly Ile Ile Asn His Leu Asp Tyr Ile Gln Gly Met Gly Phe Thr Ala
       45                  50                  55
   Ile Trp Ile Ser Pro Ile Thr Glu Gln Leu Pro Gln Asp Thr Ser Asp
   60                  65                  70                  75
   Gly Glu Ala Tyr His Gly Tyr Trp Gln Gln Lys Ile Tyr Tyr Val Asn
                   80                  85                  90
   Ser Asn Phe Gly Thr Ala Asp Asp Leu Lys Ser Leu Ser Asp Ala Leu
               95                  100                 105
   His Ala Arg Gly Met Tyr Leu Met Val Asp Val Val Pro Asn His Met
           110                 115                 120
   Gly Tyr Ala Gly Asn Gly Asn Asp Val Asp Tyr Ser Val Phe Asp Pro
       125                 130                 135
   Phe Asp Ser Ser Ser Tyr Phe His Pro Tyr Cys Leu Ile Thr Asp Trp
   140                 145                 150                 155
   Asp Asn Leu Thr Met Val Gln Asp Cys Trp Glu Gly Asp Thr Ile Val
                   160                 165                 170
   Ser Leu Pro Asp Leu Asn Thr Thr Glu Thr Ala Val Arg Thr Ile Trp
               175                 180                 185
   Tyr Asp Trp Val Ala Asp Leu Val Ser Asn Tyr Ser Val Asp Gly Leu
           190                 195                 200
   Arg Ile Asp Ser Val Glu Glu Val Glu Pro Asp Phe Phe Pro Gly Tyr
       205                 210                 215
   Gln Glu Ala Ala Gly Val Tyr Cys Val Gly Glu Val Asp Asn Gly Asn
   220                 225                 230                 235
   Pro Ala Leu Asp Cys Pro Tyr Gln Lys Tyr Leu Asp Gly Val Leu Asn
                   240                 245                 250
   Tyr Pro Ile Tyr Trp Gln Leu Leu Tyr Ala Phe Glu Ser Ser Ser Gly
               255                 260                 265
   Ser Ile Ser Asn Leu Tyr Asn Met Ile Lys Ser Val Ala Ser Asp Cys
           270                 275                 280
   Ser Asp Pro Thr Leu Leu Gly Asn Phe Ile Glu Asn His Asp Asn Pro
       285                 290                 295
   Arg Phe Ala Ser Tyr Thr Ser Asp Tyr Ser Gln Ala Lys Asn Val Leu
   300                 305                 310                 315
   Ser Tyr Ile Phe Leu Ser Asp Gly Ile Pro Ile Val Tyr Ala Gly Glu
                   320                 325                 330
   Glu Gln His Tyr Ser Gly Gly Asp Val Pro Tyr Asn Arg Glu Ala Thr
               335                 340                 345
   Trp Leu Ser Gly Tyr Asp Thr Ser Ala Glu Leu Tyr Thr Trp Ile Ala
           350                 355                 360
   Thr Thr Asn Ala Ile Arg Lys Leu Ala Ile Ser Ala Asp Ser Asp Tyr
       365                 370                 375
   Ile Thr Tyr Lys Asn Asp Pro Ile Tyr Thr Asp Ser Asn Thr Ile Ala
   380                 385                 390                 395
   Met Arg Lys Gly Thr Ser Gly Ser Gln Ile Ile Thr Val Leu Ser Asn
                   400                 405                 410
   Lys Gly Ser Ser Gly Ser Ser Tyr Thr Leu Thr Leu Ser Gly Ser Gly
               415                 420                 425
   Tyr Thr Ser Gly Thr Lys Leu Ile Glu Ala Tyr Thr Cys Thr Ser Val
           430                 435                 440
   Thr Val Asp Ser Asn Gly Asp Ile Pro Val Pro Met Ala Ser Gly Leu
       445                 450                 455
   Pro Arg Val Leu Leu Pro Ala Ser Val Val Asp Ser Ser Ser Leu Cys
   460                 465                 470                 475
   Gly Gly Ser Gly Asn Thr Thr Thr Thr Thr Thr Ala Ala Thr Ser Thr
                   480                 485                 490
   Ser Lys Ala Thr Thr Ser Ser Ser Ser Ser Ser Ala Ala Ala Thr Thr
               495                 500                 505
   Ser Ser Ser Cys Thr Ala Thr Ser Thr Thr Leu Pro Ile Thr Phe Glu
           510                 515                 520
   Glu Leu Val Thr Thr Thr Tyr Gly Glu Glu Val Tyr Leu Ser Gly Ser
   525 530 535
   Ile Ser Gln Leu Gly Glu Trp His Thr Ser Asp Ala Val Lys Leu Ser
   540 545 550 555
   Ala Asp Asp Tyr Thr Ser Ser Asn Pro Glu Trp Ser Val Thr Val Ser
   560 565 570
   Leu Pro Val Gly Thr Thr Phe Glu Tyr Lys Phe Ile Lys Val Asp Glu
   575 580 585
   Gly Gly Ser Val Thr Trp Glu Ser Asp Pro Asn Arg Glu Tyr Thr Val
   590 595 600
   Pro Glu Cys Gly Ser Gly Ser Gly Glu Thr Val Val Asp Thr Trp Arg
   605 610 615
<210> 42
   <211> 505
   <212> PRT
   <213> Aspergillus niger
<220>
   <221> mat_peptide
   <222> (22)..(505)
<400> 42
   Met Arg Leu Ser Thr Ser Ser Leu Phe Leu Ser Val Ser Leu Leu Gly
   -20 -15 -10
   Lys Leu Ala Leu Gly Leu Ser Ala Ala Glu Trp Arg Thr Gln Ser Ile
   -5 -1 1 5 10
   Tyr Phe Leu Leu Thr Asp Arg Phe Gly Arg Thr Asp Asn Ser Thr Thr
   15 20 25
   Ala Thr Cys Asp Thr Gly Asp Gln Ile Tyr Cys Gly Gly Ser Trp Gln
   30 35 40
   Gly Ile Ile Asn His Leu Asp Tyr Ile Gln Gly Met Gly Phe Thr Ala
   45 50 55
   Ile Trp Ile Ser Pro Ile Thr Glu Gln Leu Pro Gln Asp Thr Ala Asp
   60 65 70 75
   Gly Glu Ala Tyr His Gly Tyr Trp Gln Gln Lys Ile Tyr Asp Val Asn
   80 85 90
   Ser Asn Phe Gly Thr Ala Asp Asp Leu Lys Ser Leu Ser Asp Ala Leu
   95 100 105
   His Ala Arg Gly Met Tyr Leu Met Val Asp Val Val Pro Asn His Met
   110 115 120
   Gly Tyr Ala Gly Asn Gly Asn Asp Val Asp Tyr Ser Val Phe Asp Pro
   125 130 135
   Phe Asp Ser Ser Ser Tyr Phe His Pro Tyr Cys Leu Ile Thr Asp Trp
   140 145 150 155
   Asp Asn Leu Thr Met Val Gln Asp Cys Trp Glu Gly Asp Thr Ile Val
   160 165 170
   Ser Leu Pro Asp Leu Asn Thr Thr Glu Thr Ala Val Arg Thr Ile Trp
   175 180 185
   Tyr Asp Trp Val Ala Asp Leu Val Ser Asn Tyr Ser Val Asp Gly Leu
   190 195 200
   Arg Ile Asp Ser Val Leu Glu Val Glu Pro Asp Phe Phe Pro Gly Tyr
   205 210 215
   Gln Glu Ala Ala Gly Val Tyr Cys Val Gly Glu Val Asp Asn Gly Asn
   220 225 230 235
   Pro Ala Leu Asp Cys Pro Tyr Gln Lys Val Leu Asp Gly Val Leu Asn
   240 245 250
   Tyr Pro Ile Tyr Trp Gln Leu Leu Tyr Ala Phe Glu Ser Ser Ser Gly
   255 260 265
   Ser Ile Ser Asn Leu Tyr Asn Met Ile Lys Ser Val Ala Ser Asp Cys
   270 275 280
   Ser Asp Pro Thr Leu Leu Gly Asn Phe Ile Glu Asn His Asp Asn Pro
   285 290 295
   Arg Phe Ala Ser Tyr Thr Ser Asp Tyr Ser Gln Ala Lys Asn Val Leu
   300 305 310 315
   Ser Tyr Ile Phe Leu Ser Asp Gly Ile Pro Ile Val Tyr Ala Gly Glu
   320 325 330
   Glu Gln His Tyr Ser Gly Gly Lys Val Pro Tyr Asn Arg Glu Ala Thr
   335 340 345
   Trp Leu Ser Gly Tyr Asp Thr Ser Ala Glu Leu Tyr Thr Trp Ile Ala
   350 355 360
   Thr Thr Asn Ala Ile Arg Lys Leu Ala Ile Ser Ala Asp Ser Ala Tyr
   365 370 375
   Ile Thr Tyr Ala Asn Asp Ala Phe Tyr Thr Asp Ser Asn Thr Ile Ala
   380 385 390 395
   Met Arg Lys Gly Thr Ser Gly Ser Gln Val Ile Thr Val Leu Ser Asn
   400 405 410
   Lys Gly Ser Ser Gly Ser Ser Tyr Thr Leu Thr Leu Ser Gly Ser Gly
   415 420 425
   Tyr Thr Ser Gly Thr Lys Leu Ile Glu Ala Tyr Thr Cys Thr Ser Val
   430 435 440
   Thr Val Asp Ser Ser Gly Asp Ile Pro Val Pro Met Ala Ser Gly Leu
   445 450 455
   Pro Arg Val Leu Leu Pro Ala Ser Val Val Asp Ser Ser Ser Leu Cys
   460 465 470 475
   Gly Gly Ser Gly Arg Leu Tyr Val Glu
   480
<210> 43
   <211> 476
   <212> PRT
   <213> Aspergillus oryzae
<220>
   <221> mat_peptide
   <222> (1)..(476)
<400> 43
   Ala Thr Pro Ala Asp Trp Arg Ser Gln Ser Ile Tyr Phe Leu Leu Thr
   1 5 10 15
   Asp Arg Phe Ala Arg Thr Asp Gly Ser Thr Thr Ala Thr Cys Asn Thr
   20 25 30
   Ala Asp Gln Lys Tyr Cys Gly Gly Thr Trp Gln Gly Ile Ile Asp Lys
           35                  40                  45
   Leu Asp Tyr Ile Gln Gly Met Gly Phe Thr Ala Ile Trp Ile Thr Pro
       50                  55                  60
   Val Thr Ala Gln Leu Pro Gln Thr Thr Ala Tyr Gly Asp Ala Tyr His
   65                  70                  75                  80
   Gly Tyr Trp Gln Gln Asp Ile Tyr Ser Leu Asn Glu Asn Tyr Gly Thr
                   85                  90                  95
   Ala Asp Asp Leu Lys Ala Leu Ser Ser Ala Leu His Glu Arg Gly Met
               100                 105                 110
   Tyr Leu Met Val Asp Val Val Ala Asn His Met Gly Tyr Asp Gly Ala
           115                 120                 125
   Gly Ser Ser Val Asp Tyr Ser Val Phe Lys Pro Phe Ser Ser Gln Asp
       130                 135                 140
   Tyr Phe His Pro Phe Cys Phe Ile Gln Asn Tyr Glu Asp Gln Thr Gln
   145                 150                 155                 160
   Val Glu Asp Cys Trp Leu Gly Asp Asn Thr Val Ser Leu Pro Asp Leu
                   165                 170                 175
   Asp Thr Thr Lys Asp Val Val Lys Asn Glu Trp Tyr Asp Trp Val Gly
               180                 185                 190
   Ser Leu Val Ser Asn Tyr Ser Ile Asp Gly Leu Arg Ile Asp Thr Val
           195                 200                 205
   Lys His Val Gln Lys Asp Phe Trp Pro Gly Tyr Asn Lys Ala Ala Gly
       210                 215                 220
   Val Tyr Cys Ile Gly Glu Val Leu Asp Gly Asp Pro Ala Tyr Thr Cys
   225                 230                 235                 240
   Pro Tyr Gln Asn Val Met Asp Gly Val Leu Asn Tyr Pro Ile Tyr Tyr
                   245                 250                 255
   Pro Leu Leu Asn Ala Phe Lys Ser Thr Ser Gly Ser Met Asp Asp Leu
               260                 265                 270
   Tyr Asn Met Ile Asn Thr Val Lys Ser Asp Cys Pro Asp Ser Thr Leu
           275                 280                 285
   Leu Gly Thr Phe Val Glu Asn His Asp Asn Pro Arg Phe Ala Ser Tyr
       290                 295                 300
   Thr Asn Asp Ile Ala Leu Ala Lys Asn Val Ala Ala Phe Ile Ile Leu
   305                 310                 315                 320
   Asn Asp Gly Ile Pro Ile Ile Tyr Ala Gly Gln Glu Gln His Tyr Ala
                   325                 330                 335
   Gly Gly Asn Asp Pro Ala Asn Arg Glu Ala Thr Trp Leu Ser Gly Tyr
               340                 345                 350
   Pro Thr Asp Ser Glu Leu Tyr Lys Leu Ile Ala Ser Ala Asn Ala Ile
           355                 360                 365
   Arg Asn Tyr Ala Ile Ser Lys Asp Thr Gly Phe Val Thr Tyr Lys Asn
       370                 375                 380
   Trp Pro Ile Tyr Lys Asp Asp Thr Thr Ile Ala Met Arg Lys Gly Thr
   385                 390                 395                 400
   Asp Gly Ser Gln Ile Val Thr Ile Leu Ser Asn Lys Gly Ala Ser Gly
                   405                 410                 415
   Asp Ser Tyr Thr Leu Ser Leu Ser Gly Ala Gly Tyr Thr Ala Gly Gln
               420                 425                 430
   Gln Leu Thr Glu Val Ile Gly Cys Thr Thr Val Thr Val Gly Ser Asp
           435                 440                 445
   Gly Asn Val Pro Val Pro Met Ala Gly Gly Leu Pro Arg Val Leu Tyr
       450                 455                 460
   Pro Thr Glu Lys Leu Ala Gly Ser Lys Ile Cys Ser
   465                 470                 475

## Description

### FIELD OF THE INVENTION

The present invention relates, inter alia, to an enzyme comprising a carbohydrate-binding module ("CBM") and an alpha-amylase catalytic domain. The enzyme may be a hybrid between a carbohydrate-binding module ("CBM") and an alpha-amylase or the enzyme may be a variant of a parent enzyme comprising a carbohydrate-binding module ("CBM") and an alpha-amylase catalytic domain. The invention also relates to the use of the enzyme in a starch liquefaction process in which starch is degraded to smaller oligo- and/or polysaccharide fragments.

### BACKGROUND OF THE INVENTION

A large number of enzymes and processes have been described for converting starch to starch hydrolysates, such as maltose, glucose or specialty syrups, either for use as sweeteners or as precursors for other saccharides such as fructose. Glucose may also be fermented to ethanol or other fermentation products, such as citric acid, monosodium glutamate, gluconic acid, sodium gluconate, calcium gluconate, potassium gluconate, glucono delta lactone, or sodium erythorbate, itaconic acid, lactic acid, gluconic acid; ketones; amino acids, glutamic acid (sodium monoglutaminate), penicillin, tetracyclin; enzymes; vitamins, such as riboflavin, B12, beta-carotene or hormones.

Starch is a high molecular-weight polymer consisting of chains of glucose units. It usually consists of about 80% amylopectin and 20% amylose. Amylopectin is a branched polysaccharide in which linear chains of alpha-1,4 D-glucose residues are joined by alpha-1,6 glucosidic linkages.

Amylose is a linear polysaccharide built up of D-glucopyranose units linked together by alpha-1,4 glucosidic linkages. In the case of converting starch into a soluble starch hydrolysate, the starch is depolymerized. The conventional depolymerization process consists of a gelatinization step and two consecutive process steps, namely a liquefaction process and a saccharification process.

Granular starch consists of microscopic granules, which are insoluble in water at room temperature. When an aqueous starch slurry is heated, the granules swell and eventually burst, dispersing the starch molecules into the solution. During this "gelatinization" process there is a dramatic increase in viscosity. As the solids level is 30-40% in a typical industrial process, the starch has to be thinned or "liquefied" so that it can be handled. This reduction in viscosity is today mostly obtained by enzymatic degradation. During the liquefaction step, the long-chained starch is degraded into smaller branched and linear units (maltodextrins) by an alpha-amylase. The liquefaction process is typically carried out at about 105-110°C for about 5 to 10 minutes followed by about 1-2 hours at about 95°C. The temperature is then lowered to 60°C, a glucoamylase or a beta-amylase and optionally a debranching enzyme, such as an isoamylase or a pullulanase are added, and the saccharification process proceeds for about 24 to 72 hours.

It will be apparent from the above discussion that the conventional starch conversion process is very energy consuming due to the different requirements in terms of temperature during the various steps. It is thus desirable to be able to select and/or design the enzymes used in the process so that the overall process can be performed without having to gelatinize the starch. Such processes are the subject for the patents US4591560, US4727026 and US4009074 and EP0171218, and Danish patent application PA 2003 00949. The present invention discloses a new hybrid enzyme and a genetical modification of a wild type enzyme designed for such processes and comprising an amino acid sequence of a CBM and an amino acid sequence of a fungal starch degrading enzyme. Hybrid enzymes are the subject of WO9814601, WO0077165 and Danish patent application PA 2003 00949.

Kaneko et al. Journal of Fermentation and Bioengineering, Vol. 81, No 4, p. 292-298, 1996, concerns cloning and determination of the nucleotide sequence of a gene encoding an acid-stable alpha-amylase derived from *Aspergillus kawachii* comprising a catalytic module and a carbohydrate binding module.

### SUMMARY OF THE INVENTION

The invention provides in a first aspect a hybrid enzyme which comprises an amino acid sequence of a catalytic module and an amino acid sequence of a carbohydrate-binding module as defined in claim 1.

In further aspects the invention provides an isolated DNA sequence encoding the hybrid enzyme of the first aspect, a DNA construct comprising the DNA sequence encoding the hybrid enzyme of the first aspect, an expression vector comprising the DNA sequence encoding the hybrid enzyme of the first aspect, and a host cell transformed with a vector; which host cell is capable of expressing the DNA sequence encoding the hybrid enzyme of the first aspect.

In a further aspect the invention provides a method for liquefying starch, wherein a gelatinized or granular starch substrate is treated in aqueous medium with the hybrid enzyme of the first aspect of the invention.

### DETAILED DESCRIPTION OF THE INVENTION

The term "granular starch" is understood as raw uncooked starch, i.e. starch that has not been subjected to a gelatinization. Starch is formed in plants as tiny granules insoluble in water. These granules are preserved in starches at temperatures below the initial gelatinization temperature. When put in cold water, the grains may absorb a small amount of the liquid. Up to 50°C to 70°C the swelling is reversible, the degree of reversibility being dependent upon the particular starch. With higher temperatures an irreversible swelling called gelatinization begins.

The term "initial gelatinization temperature" is understood as the lowest temperature at which gelatinization of the starch commences. Starch heated in water begins to gelatinize between 50°C and 75°C; the exact temperature of gelatinization depends on the specific starch and can readily be determined by the skilled artisan. Thus, the initial gelatinization temperature may vary according to the plant species, to the particular variety of the plant species as well as with the growth conditions. In the context of this invention the initial gelatinization temperature of a given starch is the temperature at which birefringence is lost in 5% of the starch granules using the method described by Gorinstein. S. and Lii. C., Starch/Stärke, Vol. 44 (12) pp. 461-466 (1992).

The term "soluble starch hydrolysate" is understood as the soluble products of the processes of the invention and may comprise mono- di-, and oligosaccharides, such as glucose, maltose, maltodextrins, cyclodextrins and any mixture of these. Preferably at least 90%, 91%, 92%, 93%, 94%, 95%, 96%, 97% or 98% of the dry solids of the granular starch is converted into a soluble starch hydrolysate.

The term polypeptide "homology" is understood as the degree of identity between two sequences indicating a derivation of the first sequence from the second. The homology may suitably be determined by means of computer programs known in the art such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-453. The following settings for amino acid sequence comparison are used: GAP creation penalty of 3.0 and GAP extension penalty of 0.1. The relevant part of the amino acid sequence for the homology determination is the mature polypeptide, i.e. without the signal peptide.

### Hybrid enzymes

The present invention relates to hybrid enzymes as defined in claim 1. Enzyme classification numbers (EC numbers) referred to in the present specification with claims are in accordance with the Recommendations (1992) of the Nomenclature Committee of the International Union of Biochemistry and Molecular Biology, Academic Press Inc, 1992.

Hybrid enzymes or a genetically modified wild type enzymes as referred to herein include species comprising an amino acid sequence of an alpha-amylolytic enzyme (EC 3.2.1.1) linked (i.e. covalently bound) to an amino acid sequence comprising a carbohydrate-binding module (CBM).

CBM-containing hybrid enzymes, as well as detailed descriptions of the preparation and purification thereof, are known in the art [see, e.g. WO 90/00609, WO 94/24158 and WO 95/16782, as well as Greenwood et al. Biotechnology and Bioengineering 44 (1994) pp. 1295-1305]. They may, e.g. be prepared by transforming into a host cell a DNA construct comprising at least a fragment of DNA encoding the carbohydrate-binding module ligated, with or without a linker, to a DNA sequence encoding the enzyme of interest, and growing the transformed host cell to express the fused gene. The resulting recombinant product (hybrid enzyme) - often referred to in the art as a "fusion protein - may be described by the following general formula:

A-CBM-MR-X

In the latter formula, A-CBM is the N-terminal or the C-terminal region of an amino acid sequence comprising at least the carbohydrate-binding module (CBM) *per se.* MR is the middle region (the "linker") and X is the sequence of amino acid residues of a polypeptide encoded by a DNA sequence encodng the enzyme (or other protein) to which the CBM is to be linked.

The moiety A may either be absent (such that A-CBM is a CBM *per se,* i.e. comprises no amino acid residues other than those constituting the CBM) or may be a sequence of one or more amino acid residues (functioning as a terminal extension of the CBM *per se).* The linker (MR) may be a bond, or a short linking group comprising from about 2 to about 100 carbon atoms, in particular of from 2 to 40 carbon atoms. However, MR is preferably a sequence of from about 2 to about 100 amino acid residues, more preferably of from 2 to 40 amino acid residues, such as from 2 to 15 amino acid residues.

The moiety X may constitute either the N-terminal or the C-terminal region of the overall hybrid enzyme.

It will thus be apparent from the above that the CBM in a hybrid enzyme of the type in question may be positioned C-terminally, N-terminally or internally in the hybrid enzyme.

### Linker sequence

The linker sequence may be any suitable linker sequence. In preferred embodiments the linker sequence is derived from the *Athelia rolfsii* AMG, the *A.niger* AMG or the *A*. *kawachii alpha-*amylase such as a linker sequence selected from the list consisting of *A*. *nigerAMG* linker: T G G T T T T A T P T G S G S V T S T S K T T A T A S K T S T S T S S T S A (SEQ ID NO:26), A. *kawachii* alpha-amylase linker: T T T T T T A A A T S T S K A T T S S S S S S A A A T T S S S (SEQ ID NO:27), *Athalia rolfsii* AMG linker: G A T S P G G S S G S (SEQ ID NO:28), and the PEPT linker: P E P T P E P T (SEQ ID NO:29). In another preferred embodiment the hybrid enzymes has a linker sequence which differs from the amino acid sequence shown in SEQ ID NO:26, SEQ ID NO:27, SEQ ID NO:28, or SEQ ID NO:29 in no more than 10 positions, no more than 9 positions, no more than 8 positions, no more than 7 positions, no more than 6 positions, no more than 5 positions, no more than 4 positions, no more than 3 positions, no more than 2 positions, or even no more than 1 position.

### Carbohydrate-binding modules

A carbohydrate-binding module (CBM), or as often referred to, a carbohydrate-binding domain (CBD), is a polypeptide amino acid sequence which binds preferentially to a poly- or oligosaccharide (carbohydrate), frequently - but not necessarily exclusively - to a water-insoluble (including crystalline) form thereof.

CBMs derived from starch degrading enzymes are often referred to as starch-binding modules or SBMs (CBMs which may occur in certain amylolytic enzymes, such as certain glucoamylases, or in enzymes such as cyclodextrin glucanotransferases, or in alpha-amylases). Likewise, other sub-classes of CBMs would embrace, e.g. cellulose-binding modules (CBMs from cellulolytic enzymes), chitin-binding modules (CBMs which typically occur in chitinases), xylan-binding modules (CBMs which typically occur in xylanases), mannan-binding modules (CBMs which typically occur in mannanases). SBMs are often referred to as SBDs (Starch Binding Domains)

CBMs are found as integral parts of large polypeptides or proteins consisting of two or more polypeptide amino acid sequence regions, especially in hydrolytic enzymes (hydrolases) which typically comprise a catalytic module containing the active site for substrate hydrolysis and a carbohydrate-binding module (CBM) for binding to the carbohydrate substrate in question. Such enzymes can comprise more than one catalytic module and one, two or three CBMs, and optionally further comprise one or more polypeptide amino acid sequence regions linking the CBM(s) with the catalytic module(s), a region of the latter type usually being denoted a "linker". Examples of hydrolytic enzymes comprising a CBM - some of which have already been mentioned above - are cellulases, xylanases, mannanases, arabinofuranosidases, acetylesterases and chitinases. CBMs have also been found in algae, e.g. in the red alga *Porphyra purpurea* in the form of a non-hydrolytic polysaccharide-binding protein.

In proteins/polypeptides in which CBMs occur (e.g. enzymes, typically hydrolytic enzymes), a CBM may be located at the N or C terminus or at an internal position.

That part of a polypeptide or protein (e.g. hydrolytic enzyme) which constitutes a CBM *per se* typically consists of more than about 30 and less than about 250 amino acid residues. The "Carbohydrate-Binding Module of Family 20" or a CBM-20 module is in the context of this invention defined as a sequence of approximately 100 amino acids having at least 45% homology to the Carbohydrate-Binding Module (CBM) of the polypeptide disclosed in figure 1 by Joergensen et al (1997) in Biotechnol. Lett. 19:1027-1031. The CBM comprises the last 102 amino acids of the polypeptide, i.e. the subsequence from amino acid 582 to amino acid 683. The numbering of Glycoside Hydrolase Families applied in this disclosure follows the concept of Coutinho, P.M. & Henrissat, B. (1999) CAZy - Carbohydrate-Active Enzymes server at URL: http://afmb.cnrs-mrs.fr/∼cazy/CAZY/index.html or alternatively Coutinho, P.M. & Henrissat, B. 1999; The modular structure of cellulases and other carbohydrate-active enzymes: an integrated database approach. In "Genetics, Biochemistry and Ecology of Cellulose Degradation", K. Ohmiya, K. Hayashi, K. Sakka, Y. Kobayashi, S. Karita and T. Kimura eds., Uni Publishers Co., Tokyo, pp. 15-23, and Bourne, Y. & Henrissat, B. 2001; Glycoside hydrolases and glycosyltransferases: families and functional modules, Current Opinion in Structural Biology 11:593-600.

Examples of enzymes which comprise a CBM are alpha-amylases, maltogenic alpha-amylases, cellulases, xylanases, mannanases, arabinofuranosidases, acetylesterases and chitinases. Further CBMs include CBMs deriving from glucoamylases (EC 3.2.1.3) or from CGTases (EC 2.4.1.19).

In this connection, techniques suitable for isolating the relevant genes are well known in the art.

Preferred for the invention is CBMs of Carbohydrate-Binding Module Family 20. CBMs of Carbohydrate-Binding Module Family 20 suitable for the invention may be derived from the alpha-amylase of *Aspergillus kawachii* (EMBL:#AB008370) as well as CBMs having at least 90% homology to the amino acid sequence of SEQ ID NO:6.

Once a nucleotide sequence encoding the substrate-binding (carbohydrate-binding) region has been identified, either as cDNA or chromosomal DNA, it may then be manipulated in a variety of ways to fuse it to a DNA sequence encoding the enzyme of interest. The DNA fragment encoding the carbohydrate-binding amino acid sequence, and the DNA encoding the enzyme of interest are then ligated with or without a linker. The resulting ligated DNA may then be manipulated in a variety of ways to achieve expression.

### Alpha-amylolytic sequence

According to the invention the catalytic domain is an alpha-amylase that may be derived from *Aspergillus nigerwhich* consists of the amino acid sequence shown in SEQ ID NO:8

A hybrid enzyme of the invention consists of the *A.niger* acid alpha-amylase catalytic module having the sequence shown in SEQ ID NO:8 and the *A. kawachii* alpha-amylase linker and CBM (SEQ ID NO:6).

### Expression vectors

The present invention also relates to recombinant expression vectors which may comprise a DNA sequence encoding the hybrid enzyme, a promoter, a signal peptide sequence, and transcriptional and translational stop signals. The various DNA and control sequences described above may be joined together to produce a recombinant expression vector which may include one or more convenient restriction sites to allow for insertion or substitution of the DNA sequence encoding the polypeptide at such sites. Alternatively, the DNA sequence of the present invention may be expressed by inserting the DNA sequence or a DNA construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector (*e.g*. a plasmid or virus), which can be conveniently subjected to recombinant DNA procedures and can bring about the expression of the DNA sequence. The choice of the vector will typically depend on the compatibility of the vector with the host cell into which the vector is to be introduced. The vectors may be linear or closed circular plasmids. The vector may be an autonomously replicating vector, *i.e*. a vector which exists as an extrachromosomal entity, the replication of which is independent of chromosomal replication, *e.g*. a plasmid, an extrachromosomal element, a minichromosome, a cosmid or an artificial chromosome. The vector may contain any means for assuring self-replication. Alternatively, the vector may be one which, when introduced into the host cell, is integrated into the genome and replicated together with the chromosome(s) into which it has been integrated. The vector system may be a single vector or plasmid or two or more vectors or plasmids which together contain the total DNA to be introduced into the genome of the host cell, or a transposon.

### Markers

The vectors of the present invention preferably contain one or more selectable markers, which permit easy selection of transformed cells. A selectable marker is a gene the product of which provides for biocide or viral resistance, resistance to heavy metals, prototrophy to auxotrophs, and the like.

Examples of selectable markers for use in a filamentous fungus host cell may be selected from the group including, but not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), and glufosinate resistance markers, as well as equivalents from other species. Preferred for use in an *Aspergillus* cell are the *amdS* and *pyrG* markers of *Aspergillus nidulans* or *Aspergillus oryzae* and the *bar* marker of *Streptomyces hygroscopicus.* Furthermore, selection may be accomplished by co-transformation, *e.g.* as described in WO 91/17243, where the selectable marker is on a separate vector.

The vectors of the present invention preferably contain an element(s) that permits stable integration of the vector into the host cell genome or autonomous replication of the vector in the cell independent of the genome of the cell.

The vectors of the present invention may be integrated into the host cell genome when introduced into a host cell. For integration, the vector may rely on the DNA sequence encoding the polypeptide of interest or any other element of the vector for stable integration of the vector into the genome by homologous or none homologous recombination. Alternatively, the vector may contain additional DNA sequences for directing integration by homologous recombination into the genome of the host cell. The additional DNA sequences enable the vector to be integrated into the host cell genome at a precise location(s) in the chromosome(s). To increase the likelihood of integration at a precise location, the integrational elements should preferably contain a sufficient number of DNAs, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding DNA sequences. On the other hand, the vector may be integrated into the genome of the host cell by non-homologous recombination. These DNA sequences may be any sequence that is homologous with a target sequence in the genome of the host cell, and, furthermore, may be non-encoding or encoding sequences.

For autonomous replication, the vector may further comprise an origin of replication enabling the vector to replicate autonomously in the host cell in question.

The episomal replicating the AMA1 plasmid vector disclosed in WO 00/24883 may be used.

More than one copy of a DNA sequence encoding a polypeptide of interest may be inserted into the host cell to amplify expression of the DNA sequence. Stable amplification of the DNA sequence can be obtained by integrating at least one additional copy of the sequence into the host cell genome using methods well known in the art and selecting for transformants.

The procedures used to ligate the elements described above to construct the recombinant expression vectors of the present invention are well known to one skilled in the art (see, e.g. Sambrook et al, 1989, Molecular Cloning, A Laboratory Manual, 2nd edition, Cold Spring Harbor, New York).

### Host cells

The host cell of the invention, either comprising a DNA construct or an expression vector comprising the DNA sequence encoding the hybrid enzyme or a genetically modified wild type enzyme, is advantageously used as a host cell in the recombinant production of the hybrid enzyme or a genetically modified wild type enzyme. The cell may be transformed with an expression vector. Alternatively, the cell may be transformed with the DNA construct of the invention encoding the hybrid enzyme or a genetically modified wild type enzyme, conveniently by integrating the DNA construct (in one or more copies) in the host chromosome. Integration of the DNA construct into the host chromosome may be performed according to conventional methods, *e.g*. by homologous or heterologous recombination.

The host cell may be any appropriate prokaryotic or eukaryotic cell, e.g. a bacterial cell, a filamentous fungus cell, a plant cell or a mammalian cell.

In a preferred embodiment, the host cell is a filamentous fungus represented by the following groups of *Ascomycota,* include, *e.g. Neurospora, Eupenicillium (=Penicillium), Emericella (=Aspergillus), Eurotium (=Aspergillus).*

In a more preferred embodiment, the filamentous fungus include all filamentous forms of the subdivision *Eumycota* and *Oomycota* (as defined by Hawksworth et al. In, Ainsworth and Bisby's Dictionary of The Fungi, 8th edition, 1995, CAB International, University Press, Cambridge, UK. The filamentous fungi are characterized by a vegetative mycelium composed of chitin, cellulose, glucan, chitosan, mannan, and other complex polysaccharides. Vegetative growth is by hyphal elongation and carbon catabolism is obligately aerobic.

In an even more preferred embodiment, the filamentous fungus host cell is a cell of a species of, but not limited to a cell selected from the group consisting of a strain belonging to a species of *Aspergillus,* preferably *Aspergillus oryzae, Aspergillus niger, Aspergillus awamori, Aspergillus kawachii,* or a strain of *Bacillus,* or a strain of *Fusarium,* such as a strain of *Fusarium oxysporium, Fusarium graminearum* (in the perfect state named *Gribberella zeae,* previously *Sphaeria zeae,* synonym with *Gibberella roseum* and *Gibberella roseum* f. sp. *cerealis*), or *Fusarium sulphureum* (in the prefect state named *Gibberella puricaris,* synonym with *Fusarium trichothecioides, Fusarium bactridioides, Fusarium sambucium, Fusarium roseum,* and *Fusarium roseum* var. *graminearum*), *Fusarium cerealis* (synonym with *Fusarium crookwellense),* or *Fusarium venenatum,*

In a most preferred embodiment, the filamentous fungus host cell is a cell of a strain belonging to a species of *Aspergillus,* preferably *Aspergillus oryzae,* or *Aspergillus niger.*

The host cell may be a wild type filamentous fungus host cell or a variant, a mutant or a genetically modified filamentous fungus host cell. In a preferred embodiment of the invention the host cell is a protease deficient or protease minus strain. Also specifically contemplated is *Aspergillus* strains, such as Aspergillus *niger* strains, genetically modified to disrupt or reduce expression of glucoamylase, acid-stable alpha-amylase, alpha-1,6 transglucosidase, and protease activities.

### Transformation of filamentous fungus host cells

Filamentous fungus host cells may be transformed by a process involving protoplast formation, transformation of the protoplasts, and regeneration of the cell wall in a manner known in the art. Suitable procedures for transformation of *Aspergillus* host cells are described in EP 238 023, EP 184 438, and Yelton et al. 1984, Proceedings of the National Academy of Sciences USA 81:1470-1474. A suitable method of transforming *Fusarium* species is described by Malardier et al. 1989, Gene 78:147-156 or US 6,060,305.

### Isolating and cloning a DNA sequence encoding a parent alpha-amylase

The techniques used to isolate or clone a DNA sequence encoding a polypeptide of interest are known in the art and include isolation from genomic DNA, preparation from cDNA, or a combination thereof. The cloning of the DNA sequences of the present invention from such genomic DNA can be effected, e.g. by using the well known polymerase chain reaction (PCR) or antibody screening of expression libraries to detect cloned DNA fragments with shared structural features. See, *e.g.* Innis et al., 1990, PCR: A Guide to Methods and Application, Academic Press, New York. Other DNA amplification procedures such as ligase chain reaction (LCR), ligated activated transcription (LAT) and DNA sequence-based amplification (NASBA) may be used.

The DNA sequence encoding a parent alpha-amylase may be isolated from any cell or microorganism producing the alpha-amylase in question, using various methods well known in the art. First, a genomic DNA and/or cDNA library should be constructed using chromosomal DNA or messenger RNA from the organism that produces the alpha-amylase to be studied. Then, if the amino acid sequence of the alpha-amylase is known, labeled oligonucleotide probes may be synthesized and used to identify alpha-amylase-encoding clones from a genomic library prepared from the organism in question. Alternatively, a labelled oligonucleotide probe containing sequences homologous to another known alpha-amylase gene could be used as a probe to identify alpha-amylase-encoding clones, using hybridization and washing conditions of very low to very high stringency.

Yet another method for identifying alpha-amylase-encoding clones would involve inserting fragments of genomic DNA into an expression vector, such as a plasmid, transforming alpha-amylase-negative bacteria with the resulting genomic DNA library, and then plating the transformed bacteria onto agar containing a substrate for alpha-amylase (*i.e*. maltose), thereby allowing clones expressing the alpha-amylase to be identified.

Alternatively, the DNA sequence encoding the enzyme may be prepared synthetically by established standard methods, *e.g*. the phosphoroamidite method described S.L. Beaucage and M.H. Caruthers, (1981), Tetrahedron Letters 22, p. 1859-1869, or the method described by Matthes et al. (1984), EMBO J. 3, p. 801-805. In the phosphoroamidite method, oligonucleotides are synthesized, *e.g*. in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al. (1988), Science 239, 1988, pp. 487-491.

### Isolated DNA sequence

The present invention relates, inter alia, to an isolated DNA sequence comprising a DNA sequence encoding a hybrid enzyme of the invention.

The term "isolated DNA sequence" as used herein refers to a DNA sequence, which is essentially free of other DNA sequences, *e.g*. at least about 20% pure, preferably at least about 40% pure, more preferably at least about 60% pure, even more preferably at least about 80% pure, and most preferably at least about 90% pure as determined by agarose electrophoresis.

For example, an isolated DNA sequence can be obtained by standard cloning procedures used in genetic engineering to relocate the DNA sequence from its natural location to a different site where it will be reproduced. The cloning procedures may involve excision and isolation of a desired DNA fragment comprising the DNA sequence encoding the polypeptide of interest, insertion of the fragment into a vector molecule, and incorporation of the recombinant vector into a host cell where multiple copies or clones of the DNA sequence will be replicated. An isolated DNA sequence may be manipulated in a variety of ways to provide for expression of the polypeptide of interest. Manipulation of the DNA sequence prior to its insertion into a vector may be desirable or necessary depending on the expression vector. The techniques for modifying DNA sequences utilizing recombinant DNA methods are well known in the art.

### DNA construct

The present invention relates, inter alia, to a DNA construct comprising a DNA sequence encoding a hybrid enzyme of the invention."DNA construct" is defined herein as a DNA molecule, either single- or double-stranded, which is isolated from a naturally occurring gene or which has been modified to contain segments of DNA, which are combined and juxtaposed in a manner, which would not otherwise exist in nature. The term DNA construct is synonymous with the term expression cassette when the DNA construct contains all the control sequences required for expression of a coding sequence of the present invention.

### Starch processing

The hybrid enzyme of the first aspect of the invention may in an aspect be used in a method for liquefying starch, wherein a gelatinized or granular starch substrate is treated in aqueous medium with the hybrid enzyme. Preferably the process comprising hydrolysis of a slurry of gelatinized or granular starch, in particular hydrolysis of granular starch into a soluble starch hydrolysate at a temperature below the initial gelatinization temperature of said granular starch.

In a preferred embodiment the starch slurry in addition to being contacted with the hybrid enzyme of the first aspect of the invention is contacted with an enzyme selected from the list consisting of; a fungal alpha-amylase (EC 3.2.1.1), a beta-amylase (E.C. 3.2.1.2), and a glucoamylase (E.C.3.2.1.3). In an embodiment further a Termamyl-like alpha-amylase or a debranching enzyme, such as an isoamylase (E.C. 3.2.1.68) or a pullulanases (E.C. 3.2.1.41) is added. In the context of the present invention a Termamyl-like alpha-amylase is an alpha-amylase as defined in WO99/19467 on page 3, line 18 to page 6, line 27.

The starch slurry to be subjected to the method of the invention may have 20-55% dry solids granular starch, preferably 25-40% dry solids granular starch, more preferably 30-35% dry solids granular starch.

After being subjected to the method of the invention at least 85%, at least 86%, at least 87%, at least 88%, at least 89%, at least 90%, at least 91 %, at least 92%, at least 93%, at least 94%, at least 95%, at least 96%, at least 97%, at least 98%, or preferably at least 99% of the dry solids of the granular starch is converted into a soluble starch hydrolysate.

According to the invention the method of the invention is conducted at a temperature below the initial gelatinization temperature. Preferably the temperature at which the processes are conducted is at least 30°C, at least 31°C, at least 32°C, at least 33°C, at least 34°C, at least 35°C, at least 36°C, at least 37°C, at least 38°C, at least 39°C, at least 40°C, at least 41 °C, at least 42°C, at least 43°C, at least 44°C, at least 45°C, at least 46°C, at least 47°C, at least 48°C, at least 49°C, at least 50°C, at least 51°C, at least 52°C, at least 53°C, at least 54°C, at least 55°C, at least 56°C, at least 57°C, at least 58°C, at least 59°C, or preferably at least 60°C.

The pH at which the methodof the invention is conducted may in be in the range of 3.0 to 7.0, preferably from 3.5 to 6.0, or more preferably from 4.0-5.0.

The granular starch to be processed in the method of the invention may in particular be obtained from tubers, roots, stems, legumes, cereals or whole grain. More specifically the granular starch may be obtained from corns, cobs, wheat, barley, rye, milo, sago, cassava, tapioca, sorghum, rice, peas, bean, banana or potatoes. Specially contemplated are both waxy and non-waxy types of corn and barley. The granular starch to be processed may be a highly refined starch quality, preferably at least 90%, at least 95%, at least 97% or at least 99.5 % pure or it may be a more crude starch containing material comprising milled whole grain including non-starch fractions such as germ residues and fibres. The raw material, such as whole grain, is milled in order to open up the structure and allowing for further processing. Two milling processes are preferred according to the invention: wet and dry milling. In dry milling the whole kernel is milled and used. Wet milling gives a good separation of germ and meal (starch granules and protein) and is with a few exceptions applied at locations where the starch hydrolysate is used in production of syrups. Both dry and wet milling is well known in the art of starch processing and are equally contemplated for the process of the invention. Also corn grits, and preferably milled corn grits may be applied.

In an embodiment of the method of the invention the hybrid enzyme is used in a process for production of fuel or potable ethanol comprising contacting the treated starch with a yeast. Preferably the process comprises fermentation with a yeast carried out simultaneously or separately/sequential to the hydrolysis of the granular starch slurry. When the fermentation is performed simultaneous to the hydrolysis the temperature is preferably between 30°C and 35°C, and more preferably between 31 °C and 34°C.

The glucose may also be fermented into other fermentation products, such as citric acid, itaconic acid, lactic acid, gluconic acid; ketones; amino acids, such as glutamic acid (sodium monoglutaminate), but also more complex compounds such as antibiotics, such as penicillin, tetracyclin; enzymes; vitamins, such as riboflavin, B12, beta-carotene; hormones, which are difficult to produce synthetically.

### Dough-based products

### The hybrid enzyme of the invention may be used for the preparation of a dough-based edible product

The dough generally comprises flour (particularly wheat flour) and water. The dough is leavened e.g. by adding chemical leavening agents or yeast, usually *Saccharomyces cerevisiae* (baker's yeast).

The dough-based product is made by leavening and heating the dough, e.g. by baking or steaming. Examples are steamed or baked bread (in particular white, whole-meal or rye bread), typically in the form of loaves or rolls,

The dough may comprise one or more additional enzymes, e.g. a second amylase (e.g. a maltogenic alpha-amylase), a cyclodextrin glucanotransferase, a protease or peptidase, in particular an exopeptidase, a transglutaminase, a lipase, a phospholipase, a cellulase, a hemicellulase (e.g. a pentopsanase or xylanase), a glycosyltransferase, a branching enzyme or an oxidase such as glucose oxidase or an oxidase with higher activity on maltose than on glucose.

The hybrid enzyme may be used at a lower dosage than the catalytic module with alpha-amylase activity used alone (compared on a weight basis).

### MATERIALS AND METHODS

### Purchased material

Enzymes for DNA manipulations (e.g. restriction endonucleases, ligases etc.) are obtainable from New England Biolabs, Inc. and were used according to the manufacturer's instructions. Amplified plasmids were recovered with Qiagen^{®}Plasmid Kit (Qiagen). Polymerase Chain Reaction (PCR) was carried out with Expand^{™} PCR system (Roche). QIAquick^{™} Gel Extraction Kit (Qiagen) was used for purification of PCR fragments and DNA fragments excised from agarose gels.

### Strains and plasmids

*Aspergillus kawachii* strain IFO4308 was used as donor of CBM and linker sequences. *Aspergillus niger* strain DSM 2761 donated the amylolytic sequence (WO89/01969). *Aspergillus niger* strain MBin120 described in US provisional patent application No. 60/459902 (10345.000-US) was used as host strain. The Aspergillus expression plasmid pMT2188 described in the patent WO200295014 was used as vector. The *pyrF* defective *Escherichia coli* strain DB6507 (ATCC 35673) was used for propagation of pHUda381 and pHUda387

Plasmid pMT2188 consists of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminator (Tamg). Also present on the plasmid is the *Aspergillus* selective marker *amdS* from *Aspergillus nidulans* enabling growth on acetamide as sole nitrogen source and the URA3 marker from *Saccharomyces cerevisiae* enabling growth of the *pyrF* defective *Escherichia coli strain* DB6507 (ATCC 35673), which propagates pHUda381 and pHUda387. Transformation into *E*. *coli* DB6507 using the S. *cerevisiae* URA 3 gene as selective marker was done in the following way:

*E. coli* DB6507 was made competent by the method of Mandel and Higa (Mandel, M. and A. Higa (1970) J. Mol. Biol. 45, 154). Transformants were selected on solid M9 medium (Sambrook et. al (1989) Molecular cloning, a laboratory manual, 2. edition, Cold Spring Harbor Laboratory Press) supplemented with 1 g/l casaminoacids, 500 µ/l thiamine and 10 mg/l kanamycin.

### Media and substrates

Cove was composed of 342.3 g/L Sucrose, 20 ml/L COVE salt solution, 10mM Acetamide and 30 g/L noble agar. Cove-2 was composed of 30 g/L Sucrose, 20 ml/L COVE salt solution, 10mM, Acetamide and 30 g/L noble agar. COVE salt solution was composed of 26 g/L KCI, 26 g/L MgSO₄-7H₂O, 76 g/L KH₂PO₄ and 50ml/L Cove trace metals. COVE trace metals was composed of 0.04 g/L Na₂B₄O₇·10 H₂O, 0.4 g/L CuSO₄·5H₂O, 1.2 g/L FeSO₄·7H₂O, 1.0 g/L MnSO₄·H₂O, 0.8 g/L Na₂MoO₂·2H₂O and 10 g/L ZnSO₄·7H₂O- YPG was composed of 4 g/L yeast extract, 1 g/L K₂HPO₄, 0.5 g/L MgSO₄·7H₂O and 15 g/L glucose, pH 6.0. STC was composed of 0.8 M Sorbitol, 25 mM Tris pH 8 and 25 mM CaCl₂. STPC was composed of 40 % PEG4000 in STC buffer. Cove top agarose was composed of 342.3 g/L Sucrose, 20 ml/L COVE salt solution, 10mM Acetamide and 10 g/L low melt agarose. MLC was composed of 40 g/L Glucose, 50 g/L Soybean powder, 4 g/L Citric acid, pH 5.0. GO-50 was composed of glucose 50 g/L, KH2PO4 2 g/L, MgSO4-7aq 2 g/L, K2SO4 3 g, citric acid 3 g/L, oxalic acid 50 g/L, AMG trace metal solution 0.5 m/L and urea 3 g/L, pH 5.0. AMG trace metal solution was composed of 6.8 g/L ZnCl₂·7H₂O, 2.5 g/L CuSO₄·5H₂O, 0.24 g/L NiCl₂·6H₂O, 13.9 g/L FeSO₄·7H₂O, 13.5 g/L MnSO₄·H₂O and 3 g/L citric acid.

### Acid stable alpha-amylase activity

When used according to the present invention the activity of any acid stable alpha-amylase may be measured in AFAU (Acid Fungal Alpha-amylase Units), which are determined relative to an enzyme standard. 1 FAU is defined as the amount of enzyme which degrades 5.260 mg starch dry matter per hour under the below mentioned standard conditions.

Acid stable alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions. 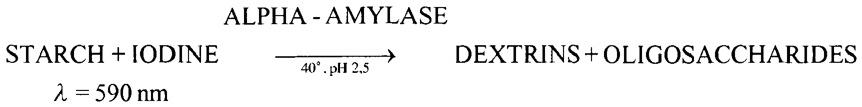 blue/violet t = 23 sec. decoloration

### Standard conditions/reaction conditions:

| | |
|---|---|
| Substrate: | Soluble starch, approx. 0.17 g/L |
| Buffer: | Citrate, approx. 0.03 M |
| Iodine (l2): | 0.03 g/L |
| CaCl2: | 1.85 mM |
| pH: | 2.50 ± 0.05 |
| Incubation temperature: | 40°C |
| Reaction time: | 23 seconds |
| Wavelength: | 590nm |
| Enzyme concentration: | 0.025 AFAU/mL |
| Enzyme working range: | 0.01-0.04 AFAU/mL |

A folder EB-SM-0259.02/01 describing this analytical method in more detail is available upon request to Novozymes A/S, Denmark, which folder is hereby included by reference.

### Glucoamylase activity

Glucoamylase activity may be measured in AmyloGlucosidase Units (AGU). The AGU is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

### AMG incubation:

| | |
|---|---|
| Substrate: | maltose 23.2 mM |
| Buffer: | acetate 0.1 M |
| pH: | 4.30 ± 0.05 |
| Incubation | 37°C ± 1 |
| temperature: | |
| Reaction time: | 5 minutes |
| Enzyme working range: | 0.5-4.0 AGU/mL |

### Color reaction:

| | |
|---|---|
| GlucDH: | 430 U/L |
| Mutarotase: | 9 U/L |
| NAD: | 0.21 mM |
| Buffer: | phosphate 0.12 M; 0.15 M NaCl |
| pH: | 7.60 ± 0.05 |
| Incubation | 37°C ± 1 |
| temperature: | |
| Reaction time: | 5 minutes |
| Wavelength: | 340 nm |

A folder (EB-SM-0131.02/01) describing this analytical method in more detail is available on request from Novozymes A/S, Denmark, which folder is hereby included by reference.

### DNA manipulations

Unless otherwise stated, DNA manipulations and transformations were performed using standard methods of molecular biology as described in Sambrook et al. (1989) Molecular cloning: A laboratory manual, Cold Spring Harbor lab. Cold Spring Harbor, NY; Ausubel, F. M. et al. (eds.) "Current protocols in Molecular Biology", John Wiley and Sons, 1995; Harwood, C. R. and Cutting, S. M. (eds.)

### Example 1: Cloning of carbohydrate binding module (CBM) from Aspergillus kawachii

In order to clone the carbohydrate binding module (CBM) with linker from *A.kawachii,* the primers CBM1 (SEQ ID NO:1) and CBM2 (SEQ ID NO:2) were designed based on the nucleotide sequences of *Aspergillus kawachii* acid stable alpha-amylase in the EMBL database (EMBL:#AB008370). CBM1 and CBM2 comprise a BamHI site and a SalI site, respectively.
CBM1: 5'- gaagggatccgatttttactagtacatccaaagccaccac- 3'
CBM2: 5'- tttgtcgacctacctccacgtatcaaccaccgtctcc- 3'

PCR reaction was carried out with the primers CBM1 and CBM2 using genomic DNA from *A. kawachii* (IFO4308) as template. Reaction components (1 ng /microL of genomic DNA, 250 mM dNTP each, primer 250 nM each, 0.1 U/ microL in Taq polymerase in 1X buffer (Roche Diagnostics, Japan)) were mixed and submitted for PCR under the following conditions.

| Step | Temperature | Time |
|---|---|---|
| 1 | 94°C | 2 min |
| 2 | 92°C | 1 min |
| 3 | 55°C | 1 min |
| 4 | 72°C | 1 min |
| 5 | 72°C | 10 min |
| 6 | 4°C | forever |

| | | |
|---|---|---|
| Steps 2 to 4 were repeated 30 times. | | |

A 0.4 kb fragment comprising the CBM with linker region was amplified. The amplified DNA was cut by Sal I and BamH I and ligated into pMT2188 digested by Xho I and BamH I to create pHUda381 having CBM with linker region from *Aspergillus kawachii, Aspergillus niger* neutral amylase promoter, *Aspergillus nidulans* TPI leader sequences, *Aspergillus niger* glucoamylase terminator and *Aspergillus nidulans* amdS gene as a marker. The pHUda381 was sequenced to confirm presence of correct CBM with linker sequence. The CBM with linker sequence is shown in SEQ ID NO:5.

### Example 2: Expression of the hybrid enzyme in Aspergillus niger

The production of the cDNA sequence of *Aspergillus niger* acid stable alpha-amylase gene and the cDNA clone of *Aspergillus niger* acid stable alpha-amylase are described in WO 8901969 (example 1 and 3). A PCR reaction with cDNA clone of *Aspergillus niger* acid stable alpha-amylase as template was performed using the primers (SEQ ID NO:3) and (SEQ ID NO:4) to introduce a BamHI site and a Spel site, respectively.
(SEQ ID NO:3): 5'- tttggatccaccatgagattatcgacttcgagtctcttc- 3'
(SEQ ID NO:4): 5'- tttactagtagcagcagcagttgtggtcgtggttgttc- 3'

Reaction components (1 ng /microL of template DNA, 250 mM dNTP each, primer 250 nM each, 0.1 U/ microL in Taq polymerase in 1X buffer (Roche Diagnostics, Japan)) were mixed and submitted for PCR under the following conditions.

| Step | Temperature | Time |
|---|---|---|
| 1 | 94°C | 2 min |
| 2 | 92°C | 1 min |
| 3 | 55°C | 1 min |
| 4 | 72°C | 2 min |
| 5 | 72°C | 10 min |
| 6 | 4°C | forever |

| | | |
|---|---|---|
| Steps 2 to 4 were repeated 30 times. | | |

The 1.5 kb amplified DNA fragment was cut by Spe I and BamH I and ligated into pHUda381 digested by Spe I and BamH I to create the expression plasmid pHUda387 comprising *Aspergillus niger* acid stable alpha-amylase cDNA fused with CBM from *Aspergillus kawachii.* The pHUda387 was sequenced to confirm that no changes had happen in the *Aspergillus niger acid* stable alpha-amylase cDNA sequences. The *Aspergillus niger acid* stable alpha-amylase cDNA sequence is shown in SEQ ID NO:7.

The pHUda387 was transformed into *Aspergillus niger* MBin120. The host strain was propagated in 100 ml of non-selective YPG medium at 32°C for 16 hrs on a rotary shaker at 120 rpm. Cells were collected by filtering, washed with 0.6 M KCI and resuspended in 20 ml of 0.6 M KCI containing a commercial beta-glucanase product (GLUCANEX^{™}, Novozymes A/S) at final concentration of 600 microL /ml. The suspension was incubated at 32°C at 80 rpm until protoplasts were formed, then washed twice with STC buffer. The protoplasts were counted with a hematometer and resuspended and adjusted in an 8:2:0.1 solution of STC:STPC:DMSO to a final concentration of 2.5x10⁷ protoplasts/ml. About 3 microgram of plasmid DNA was added to 100 microL of protoplast suspension, mixed gently and incubated on ice for 20 min. One ml of SPTC was added and the protoplast suspension was incubated for 30 min at 37°C. After the addition of 10 ml of 50 °C Cove top agarose, the reaction was poured onto Cove agar plates and the plates were incubated at 32°C. After 5 days transformants were selected from the Cove medium.

The selected transformants were inoculated in 100 ml of MLC media and cultivated at 30 °C for 2 days. 10 ml of MLC medium was inoculated to 100 ml of GO-50 medium and cultivated at 30°C for 5 days. The supernatant was obtained by centrifugation.

The acid stable alpha-amylase activity in the supernatant was determined as decrease of blue color of starch-iodine complex measured in OD590nm. 25 microL of enzyme samples dissolved in sample buffer (51.4 mM calcium chloride in 2 mM citrate buffer [pH 2.5]) was mixed with 135 microL of substrate solution (0.6 g/L of starch [Merck 1253] and 12 g/L of sodium acetate in 100 mM citrate buffer [pH 2.5]) and incubated at 37°C for 325 sec. After 325 sec, 90 microL of iodine solution (1.2 g/L of potassium iodine [Merck 5043] and 0.12 g/L of iodine [Merck 4761]) was added to the reaction mixture and incubated at 37°C for 25 sec. Activity was measured at 590 nm on a spectrophotometer. 25 microL of distilled water was used instead of enzyme samples in blank experiments.

### Example 3: Performance of the hybrid enzyme in SSF of non-gelatinized starch

The relative performance of *Aspergillus niger* acid stable alpha-amylase and *Aspergillus niger* acid stable alpha-amylase with an attached carbohydrate-binding module was evaluated via mini-scale SSF (Simultaneous Saccharification and Fermentation) fermentations. Dosages used were 0.3, 0.5 and 1.0 AFAU/g DS of the respective alpha-amylase complemented with a 0.5 AGU/g DS dose of purified Aspergillus *niger* glucoamylase. Briefly, approximately 1.9 g of ground corn (yellow #2 dent corn ground in a pilot scale hammer mill and passed through a 2 mm screen, 11.2% moisture content) was added to 16 ml polystyrene tubes (Falcon 352025). A solution of 0.02 N H₂SO₄ with 3 mg/ml penicillin was added in an amount appropriate to bring the dry solids level (DS) to 34% and the pH to 5.0. Treatments were made in six replicates.

After dosing the enzymes, the tubes were inoculated with 4.74 x 10⁸ yeast cells/ml. Tubes were capped with a screw on lid which had been punctured with a very small needle to allow gas release and vortexed briefly before weighing and incubation at 32°C. Fermentation progress was followed by weighing the tubes over time. Tubes were vortexed briefly before weighing. The relationship used between amount of CO₂ loss and the weight of ethanol was: *CO₂loss (g) x 1.045 = EtOH (g).* The results are shown in table 2.

**Table 2. Average ethanol yield as g ethanol/g DS after 50 hrs.**

| Dose (AFAU/g DS) | Aspergillus niger alpha-amylase | Aspergillus niger alpha-amylase+CBM |
|---|---|---|
| 0.3 | 0.342 | 0.366 |
| 0.5 | 0.348 | 0.383 |
| 1.0 | 0.365 | 0.390 |

| | | |
|---|---|---|
| 95% confidence limits:+ /- 0.008 | | |

On an equal activity basis, *Aspergillus niger* acid stable alpha-amylase with an attached carbohydrate-binding module (hybrid) significantly outperformed *Aspergillus niger* acid stable alpha-amylase (native) at all dosages tested. The hybrid enzyme was approximately three times more effective than the native enzyme (i.e. performance of *Aspergillus niger* acid stable alpha-amylase could be matched with about three times less *Aspergillus niger acid* stable alpha-amylase with an attached carbohydrate-binding module).

### Example 4: Performance of hybrid enzymes with different combinations of CBM, linker sequence and catalytic module

Hybrid enzyme variants comprising the *Aspergillus niger acid* stable alpha-amylase (AA) and a CBM from *A*. *kawachii* alpha-amylase, *A*. *niger* AMG, T. *emersonii* AMG, *Athelia rolfsii* AMG, or *Bacillus* sp. maltogenic alpha-amylase (Novamyl) were constructed. The linker sequence from *A*. *kawachii* alpha-amylase (SEQ ID NO:27) was used in all the variants.

The performance of the variants was assessed using corn starch (SIGMA S-9679) as substrate, *Aspergillus niger* glucoamylase G2 0.5 AGU/g DS, variant 1.0 AFAU/g DS and quantification of the liberated glucose with the Glucose B-test kit (Wako Pure Chemicals 271-3141). The results are shown in table 3.

| Table 3. Performance of hybrid enzymes with different CBMs. Glucose mg/ml after 20, 48, and 68 hrs (1.0 AFAU/gDS) | | | | | | |
|---|---|---|---|---|---|---|
| Variant | Catalytic module | Linker | SBD | 20 hrs | 48 hrs | 68 hrs |
| JA001 | Aspergillus niger | kawachii AA | kawachii AA | 0.18 | 0.51 | 1.07 |
| JA002 | Aspergillus niger | kawachii AA | niger AMG | 0.38 | 1.08 | 1.88 |
| JA003 | Aspergillus niger | kawachii AA | emersonii AMG | 0.04 | 0.19 | 0.43 |
| JA004 | Aspergillus niger | kawachii AA | rolfsii AMG | 0.48 | 1.43 | 2.48 |
| JA005 | Aspergillus niger | kawachii AA | bacillus MA | -0.03 | -0.04 | 0.10 |

Hybrid enzyme variants comprising the *Aspergillus niger* acid stable alpha-amylase and a CBM from *A*. *kawachii* alpha-amylase, *A. niger* AMG, or *Athelia rolfsii* AMG, were constructed. Also variants with different linker sequence were constructed. The linker sequences used were *A*. *niger* AMG linker (SEQ ID NO:26), *A. kawachii* alpha-amylase linker (SEQ ID NO:27), *Athelia rolfsii AMG* linker (SEQ ID NO:28), and the PEPT linker (SEQ ID NO:29). A variant having two CBDs in tandem was also constructed (JA012).

The performance of the variants was assessed as above, only with *Aspergillus niger* glucoamylase G1 and the variant was dosed as 0.3 AFAU/g DS. The results are shown in table 4.

| Table 4. Performance of hybrid enzymes with different linker sequences and catalytic modules. Glucose mg/ml after 18, 24, and 42 hrs. | | | | | | | |
|---|---|---|---|---|---|---|---|
| Variant | Catalytic module | Linker | SBD | 18 hrs | 24 hrs | 42 hrs | 68 hrs |
| JA001 | Aspergllus niger | kawachii AA | Kawachii AA | 2.73 | 4.40 | 7.01 | 8.93 |
| JA002 | Aspergillus niger | kawachii AA | niger AMG | 2.75 | 4.30 | 7.03 | 9.23 |
| JA004 | Aspergillus niger | kawachii AA | rolfsii AMG | 2.91 | 4.35 | 7.16 | 9.11 |
| JA008 | Aspergillus niger | niger AMG | niger AMG | 3.01 | 4.26 | 7.31 | 8.76 |
| JA009 | Aspergillus niger | rolfsii AMG | niger AMG | 3.02 | 4.69 | 7.60 | 9.11 |
| JA010 | Aspergillus niger | PEPT | niger AMG | 3.18 | 4.66 | 7.82 | 9.38 |
| JA011 | Aspergillus niger | rolfsii AMG | rolfsii AMG | 3.31 | 4.62 | 7.49 | 9.60 |
| JA012 | Aspergillus niger | kawachii AA | niger AMG +rolfsii AMG | 2.99 | 4.10 | 6.97 | 8.85 |

Hybrid enzyme variants comprising the linker and CBM from *A*. *kawachii* alpha-amylase with different catalytic modules were constructed. The catalytic modules applied were from *Aspergillus niger* acid stable alpha-amylase, *A*. *kawachii* alpha-amylase or *Aspergillus oryzae* alpha-amylase (Fungamyl^{™}). The performance of the variants was assessed as above, with *Aspergillus niger* glucoamylase G1 and the variant dosed as 0.3 AFAU/g DS. The results are shown in table 5.

| Table 5. Performance of hybrid enzymes with different catalytic modules. Glucose mg/ml after 18, 24, 42 and 68 hrs. | | | | | | | |
|---|---|---|---|---|---|---|---|
| Variant | catalytic module | Linker | SBD | 18 hrs | 24 hrs | 42 hrs | 68 hrs |
| JA001 | A. niger AA | kawachii AA | kawachii AA | 2.73 | 4.40 | 7.01 | 8.93 |
| JA006 | A. oryzae AA | kawachii AA | kawachii AA | 3.39 | 5.10 | 7.99 | 9.77 |
| JA007 | A. kawachii AA | kawachii AA | kawachii AA | 2.91 | 3.99 | 6.82 | 8.57 |

Hybrid enzyme variants comprising the linker and CBM from *A*. *kawachii* alpha-amylase or *A. rolfsii* AMG with different catalytic modules from *Aspergillus niger* acid stable alpha-amylase or Aspergillus *oryzae* alpha-amylase (Fungamyl^{™}). The performance of the variants was assessed as above with *Aspergillus niger* glucoamylase G1 and the variant dosed as 0.3 AFAU/g DS. The results are shown in table 6.

| Table 6. Performance of different hybrid enzymes. Glucose mg/ml after 18, 24 or 42 hrs. | | | | | | |
|---|---|---|---|---|---|---|
| Variant | catalytic module | Linker | SBD | 18 hrs | 24 hrs | 42 hrs |
| JA001 | A. niger AA | kawachii AA | kawachii AA | 4.64 | 5.61 | 8.38 |
| JA006 | A. oryzae AA | kawachii AA | kawachii AA | 6.27 | 7.93 | 9.40 |
| JA017 | A. oryzae AA | rolfsii AMG | rolfsii AMG | 6.68 | 8.87 | 9.80 |

### Example 5: Variants of JA017 with one or more substitutions in the catalytic module.

Variants of the hybrid enzyme JA017 (amino acid sequence shown in SEQ ID NO:40) comprising the *A. rolfsii* AMG CBM, *A. rolfsii* linker sequence and the *A*. *oryzae* alpha amylase catalytic module with one or more substitutions in the catalytic module were constructed using conventional protein engineering techniques. Table 7 lists the substitutions in the variants.

**Table 7. Variants of JA017 (SEQ ID NO:40) with one or more substitutions in the catalytic module.**

| **No.** | **Substitution** |
|---|---|
| JA019 | Y175W E176D |
| JA050 | N264K M266L |
| JA056 | D253N |
| JA057 | K158D S161 D Q163S D164S G466D D468S N470D |
| JA059 | G60N N264K M266L |
| JA060 | D177N |
| JA061 | K158D S161DQ163S D164S Y175W E176D G466D D468S N470D |
| JA062 | K158D S161DQ163S D164S Y175W E176D N264K M266L G466D D468S N470D |
| JA063 | K158V S161DQ163S D164S G466D D468S N470D |
| JA069 | K158D S161NQ163A D164S G466D D468S N470D |
| JA074 | K158V S161N Q163A D164S N264K M266L G466D D468S N470D |
| JA076 | Q81R K158V S161N Q163A D164S Y175W E176D G466D D468S N470D |
| JA083 | K158D S161DQ163S D164S Y175W E176D N264E M266L G466D D468S N470D |
| JA085 | Q81 R K158V S161N Q163A D164S Y175W E176D D177N N264K M266L G466D D468S N470D |
| JA093 | Q81 R K158V S161N Q163A D164S Y175W E176D D177N N264E M266L G466D D468S N470D |
| JA094 | K158V S161N Q163A D164S Y175W E176D D177N N264K M266L G466D D468S N470D |
| JA095 | K158V S161N Q163A D164S Y175W E176D D177N N264E M266L G466D D468S N470D |
| JA096 | K158V S161N Q163A D164S D177N N264K M266L G466D D468S N470D |
| JA097 | K158V S161N Q163A D164S D177N N264E M266L G466D D468S N470D |

The variants were expressed in *Aspergillus oryzae* and characterized for pH stability, thermostability and the performance toward raw starch by glucose releasing test.

JA085 showed at least by 5 °C higher thermostability than JA017 at pH 4.5 was and stable even at pH 3.0.

The new variants JA085 and JA074 showed a high specific activity in the glucose release test. JA085 continued to release glucose even at pH 3.8 where JA017 was quickly inactivated. JA074 had the highest glucose yields at pH 4.0 was but was inactivated at pH 3.8.

Tables 8-10 list the temperature stability as residual activity after 30 min incubation at 55°C and 60°C at pH 4.5, the specific activity, the pH stability and the activity in the glucose releasing test of a number of variants.

**Table 8. Temperature stability and specific activity of selected PE variants.**

| | Temperature stability (%) | | | Specific activity (mFAU/OD280) |
|---|---|---|---|---|
| | In 1mM CaCl₂ | | No CaCl₂ | |
| | 55C° | 60°C | 55°C | |
| JA017 | 2 | 0 | 0 | 3157 |
| JA057 | 12 | 0 | 0 | |
| JA061 | 51 | 0 | 16 | |
| JA063 | 18 | 0 | 0 | |
| JA069 | 16 | 0 | 0 | |
| JA074 | 19 | 0 | 0 | |
| JA085 | 68 | 20 | 12 | 3653 |
| JA095 | 59 | 7 | 2 | 4240 |
| JA096 | 53 | 0 | 0 | |
| JA097 | 40 | 0 | 0 | |

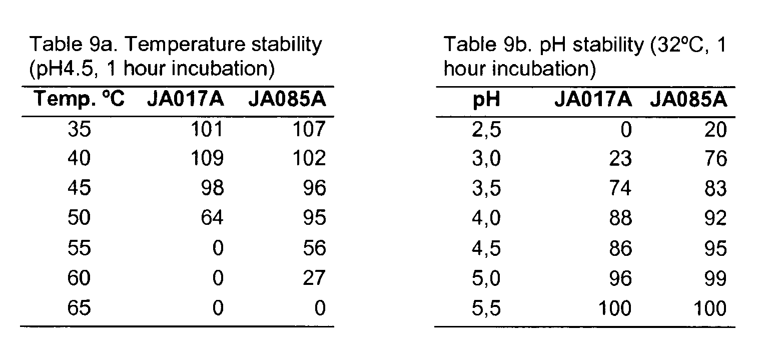

**Table 10a. Performance in glucose releasing test with purified A.niger AMG G2 at pH 4.0. Glucose mg/ml.**

| | **0 min** | **19 min** | **43 min** | **67 min** | **91 min** |
|---|---|---|---|---|---|
| JA001 | 0.48 | 3.48 | 5.67 | 8.26 | 10.79 |
| JA017 | 0.46 | 6.45 | 7.97 | 8.71 | 8.98 |
| JA074 | 0.44 | 8.85 | 14.69 | 18.24 | 20.29 |
| JA085 | 0.41 | 5.87 | 10.62 | 13.92 | 17.34 |

**Table 10b. Performance in glucose releasing test with purified A.niger AMG G2 at pH 3.8. Glucose mg/ml.**

| | **0 min** | **19 min** | **43 min** | **67 min** | **91 min** |
|---|---|---|---|---|---|
| JA001 | 0.48 | 3.19 | 5.28 | 7.63 | 10.32 |
| JA017 | 0.46 | 4.28 | 4.71 | 4.98 | 5.13 |
| JA074 | 0.43 | 7.75 | 11.58 | 13.55 | 13.93 |
| JA085 | 0.41 | 5.71 | 9.73 | 12.99 | 15.76 |

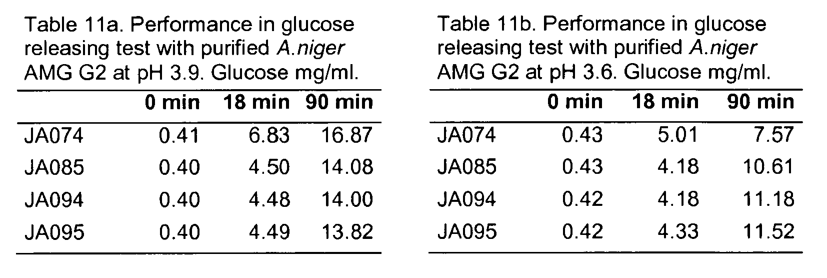

### Example 6: Variants of the Aspergillus kawachii acid alpha-amylase

A variant of the *Aspergillus kawachii* acid alpha-amylase suitable for raw starch hydrolysis may be produced by conventional protein engineered of the sequence comprising the catalytic module. Specific position wherein alterations can improve specific activity and/or stability was predicted based on similarity to one of the following fungal alpha-amylases having the indicated amino acid sequence and a three-dimensional structure found under the indicated identifier in the RCSB Protein Data Bank (www.rcsb.org): the *A*. *niger* acid alpha-amylase (2aaa, SEQ ID NO:42 herein) and the alpha-amylase (Taka amylase) from *Aspergillus oryzae* (6taa or 7taa, SEQ ID NO:43 herein). The two 3D models were superimposed by aligning the amino acid residues of each catalytic triad. This was done by methods known in the art based on the deviations of the three pairs of C-alpha atoms, e.g. by minimizing the sum of squares of the three deviations or by aligning so as to keep each deviation below 0.8 Å, e.g. below 0.6 Å, below 0.4 Å, below 0.3 Å or below 0.2 Å.

Alternatively, the superimposition may be done by aligning the two amino acid sequences by a conventional method and minimizing the sum of squares of the deviations of all corresponding pairs of amino acid residues. The sequence alignment may be done by conventional methods, e.g. by use the software GAP from UWGCG Version 8.

On the alignment of the three alpha-amylases from *A. kawachii, A. niger* (aaa_new) and *A. oryzae* (7taa) shown below are indicated the residues which are within 10Å from the acarbose substrate in 7taa.pdb structure. These residues are targets for alterations conferring improved specific activity.

The specific alterations for increased specific activity is one substitution or a combination of substitutions in the following positions within the "10Å distance of the substrate": 13,15,18,32-36 (i.e. 32, 33, 34, 35, 36), 61,63-64, 68-69,73-84 (e.g. 73, 74, 75, 76, 77, 78, 79, 80, 81, 82, 83, 84),117-125 (e.g. 117, 118, 119, 120, 121, 122, 123, 124, 125),152-158 (e.g. 152, 153, 154, 155, 156, 157, 158),161-162, 165-175 (e.g.165, 166, 167, 168, 169, 170, 171, 172, 173, 174, 175), 204-211 (e.g. 204, 205, 206, 207, 208, 209, 210, 211), 216,229-239 (229, 230, 231, 232, 233, 234, 235, 236, 237, 238, 239), 242, 250, 252-253, 255-257 (e.g. 255, 256, 257),259-260, 275, 292, 295-299 (e.g. 295, 296, 297, 298, 299), 304, 328, 339-344 according to the 7taa numbering in the alignment above where the N-termimal is ATPAD in 7taa (TAKA amylase). Most interesting positions are: 33, 36, 74, 75, 77, 120, 153, 154, 155, 156, 157, 158, 162, 166, 169, 170, 199, 232, 233, 235, 238, 239, 256, 257, 331, 336, 339, 340, and 342. The substitutions may be to any amino acid residue.

Variants comprising one of the following substitutions or combinations thereof may be produced: G33A, I36K, S74A, D75Y, E77D, P120A, I153D, D154N, W155Y, D156E, N157D, L158Q, Q162E, E166L, T169N, I170T, E199K, E199L, D232L, N233D, N235D, L238Y, D239T, W256Y, Q257P, E331Q, S336A, D339K, D339N, V340D, and Y342A. The most interesting variants comprise one or more of the following substitutions; S74A, E166L, E199L, D339K, and D156E. Yet more interesting is the variant having the multiple substitutions S74A/E166L/E199L.

Based on similarity between *Aspergillus kawachii* and the *A*. *niger* acid alpha-amylases the following substitutions is predicted to improve the specific activity; N31 D, S74A, Y89D, E209L, Y245V, D348K, D378A, K383A, P386A, I387F, I405V, N448S, and N480R, and a variant named WA01 comprising these substitutions can be produced.

### Example 7: Performance of a variant of the Aspergillus kawachii acid alpha-amylase

The sequences of the hybrids JA007 and JA001 described in example 4 are identical to respectively the sequence of the *A. kawachii* acid alpha-amylase (SEQ ID NO:41) and the sequence of the variant WA01 described in the previous example. As the two hybrids JA001 and JA007 have been compared in several tests the improvement in specific activity conferred by the substitutions to the variant WA01 can be very accurately predicted. Specific activity of the variant WA01 will be 1.7 to 1.8 times higher than the specific activity of the A. *kawachii* wild type amylase. The prediction is based on the data shown in table 12.

**Table 12. Performance of the hybrid JA007 (identical to A. kawachii acid alpha-amylase) and the hybrid JA001 (identical to WA01, a variant of the A. kawachii acid alpha-amylase comprising the substitutions N31 D, S74A, Y89D, E209L, Y245V, D348K, D378A, K383A, P386A, I387F, I405V, N448S, and N480R)**

| | **AFAU/ml** | **mg/ml** | **AFAU/mg** | **A280** | **AFAU/A280** |
|---|---|---|---|---|---|
| JA001 | 1.41 | 0.67 | 2.11 | 1.22 | 1.15 |
| JA007 | 0.56 | 0.46 | 1.22 | 0.89 | 0.63 |

### Example 8: Raw starch hydrolysis with an acid fungal alpha-amylase with or without CBD

This example illustrates the conversion of granular wheat starch or corn grits into glucose using acid fungal amylase without a CBM (SEQ ID NO:8) or the corresponding hybrid with a CBM (JA001). A slurry with 33% dry solids (DS) granular starch was prepared by adding 247.5 g of wheat starch or maize grits under stirring to 502.5 ml of water. The pH was adjusted with HCl to 4.5. The granular starch slurry was distributed to 100 ml blue cap flasks with 75 g in each flask. The flasks were incubated with magnetic stirring in a 60°C water bath. At zero hours the enzyme activities were dosed to the flasks; glucoamylase (200 AGU/kg DS), an acid fungal amylase (50 AFAU/kg DS) and the wild type *Aspergillus niger* acid alpha-amylase (SEQ ID NO:8) or the *Aspergillus niger* acid alpha-amylase but fused to a linker and CBM derived from A. kawachii (SEQ ID NO:8 fused to SEQ ID NO:6). The *Aspergillus niger* acid alpha-amylase w/wo CBM was dosed as 100 KNU/kg DS. Samples were withdrawn after 24, 48, 72, and 96 hours.

Total dry solids starch was determined using the following method. The starch was completely hydrolyzed by adding an excess amount of alpha-amylase (300 KNU/Kg dry solids) and placing the sample in an oil bath at 95 °C for 45 minutes. Subsequently the samples were cooled to 60°C and an excess amount of glucoamylase (600 AGU/kg DS) was added followed by incubation for 2 hours at 60°C.

Soluble dry solids in the starch hydrolysate were determined by refractive index measurement on samples after filtering through a 0.22 microM filter. The sugar profiles were determined by HPLC. The amount of glucose was calculated as DX.

The results are shown in tables 13-14 (wheat starch) and 15-16 (corn grits).

**Table 13. Soluble dry solids as percentage of total dry substance from wheat starch. Enzymes: glucoamylase, acid fungal alpha-amylase and more acid fungal alpha-amylase with the CBM or without the CBM.**

| | **24 hours** | **46 hours** | **70 hours** | **90 hours** |
|---|---|---|---|---|
| Without CBM | 81,1 | 88,6 | 89,4 | 90,7 |
| With CBM | 86,2 | 92,6 | 93,7 | 95,1 |

**Table 14. The DX of the soluble hydrolysate from wheat starch: Enzymes: glucoamylase, acid fungal amylase and more acid fungal alpha-amylase with the CBM or without the CBM.**

| | **24 hours** | **46 hours** | **70 hours** | **90 hours** |
|---|---|---|---|---|
| Without CBM | 78,1 | 84,9 | 85,6 | 86,7 |
| With CBM | 82,3 | 88,4 | 89,5 | 90,5 |

**Table 15. Soluble dry solids as percentage of total dry substance from corn grits. Enzymes: glucoamylase, acid fungal alpha-amylase and more acid fungal alpha-amylase with the CBM or without the CBM.**

| | **24 hours** | **46 hours** | **70 hours** | **90 hours** |
|---|---|---|---|---|
| Without CBM | 44,0 | 49,6 | 56,8 | 62,2 |
| With CBM | 53,6 | 59,7 | 63,8 | 68,4 |

**Table 16. The DX of the soluble hydrolysate from corn grits: Enzymes: glucoamylase, acid fungal amylase and more acid fungal alpha-amylase with the CBM or without the CBM.**

| | **24 hours** | **46 hours** | **70 hours** | **90 hours** |
|---|---|---|---|---|
| Without CBM | 42,1 | 47,3 | 53,9 | 58,8 |
| With CBM | 51,2 | 56,8 | 60,4 | 64,5 |

## Claims

1. A hybrid enzyme which comprises an amino acid sequence of a catalytic module and an amino acid sequence of a carbohydrate binding module,
a) wherein the catalytic module is an alpha-amylase sequence which consists of the amino acid sequence shown in SEQ ID NO:8; and,
b) wherein the carbohydrate binding module consists of an amino acid sequence having at least 90% identity to the amino acid sequence of SEQ ID NO:6.

2. An isolated DNA sequence encoding the hybrid enzyme according to claim 1.

3. A DNA construct comprising the DNA sequence according to claim 2.

4. An expression vector comprising the DNA sequence according to 2.

5. A host cell transformed with an expression vector according to claim 4 which host cell is capable of expressing the isolated DNA sequence of claim 2.

6. The host cell according to claim 5, which host cell is from a fungus, a bacteria, a plant or a mammal.

7. A method for liquefying starch, wherein a gelatinized or granular starch substrate is treated in aqueous medium with the hybrid enzyme according to claim 1.

8. The method according to claim 7 comprising contacting the treated starch with a yeast to produce fuel or potable ethanol.

9. The method according to claim 7 comprising fermenting the treated starch into a fermentation product, such as citric acid, monosodium glutamate, gluconic acid, sodium gluconate, calcium gluconate, potassium gluconate, glucono delta lactone, sodium erythorbate, itaconic acid, lactic acid, gluconic acid; ketones; amino acids, glutamic acid (sodium monoglutaminate), penicillin, tetracyclin; enzymes; vitamins, such as riboflavin, B12, beta-carotene or hormones.

10. A process for preparing a dough-based product, comprising adding the hybrid enzyme of claim 1 to a dough.

## Patentansprüche

1. Hybridenzym, das eine Aminosäuresequenz eines katalytischen Moduls und eine Aminosäuresequenz eines Kohlenhydrat bindenden Moduls umfasst,
(a) wobei das katalytische Modul eine alpha-Amylasesequenz ist, die aus der in SEQ ID NO: 8 gezeigten Aminosäuresequenz besteht; und
(b) wobei das Kohlenhydrat bindende Modul aus einer Aminosäuresequenz mit mindestens 90% Identität zu der Aminosäuresequenz von SEQ ID NO: 6 besteht.

2. Isolierte DNA-Sequenz, kodierend das Hybridenzym gemäß Anspruch 1.

3. DNA-Konstrukt, umfassend die DNA-Sequenz gemäß Anspruch 2.

4. Expressionsvektor, umfassend die DNA-Sequenz gemäß Anspruch 2.

5. Wirtszelle, transformiert mit einem Expressionsvektor gemäß Anspruch 4, wobei die Wirtszelle fähig ist, die isolierte DNA-Sequenz gemäß Anspruch 2 zu exprimieren.

6. Wirtszelle nach Anspruch 5, wobei die Wirtszelle aus einem Pilz, einer Bakterie, einer Pflanze oder einem Säuger ist.

7. Verfahren zum Verflüssigen von Stärke, wobei ein geliertes oder granuläres Stärkesubstrat in wässrigem Medium mit dem Hybridenzym gemäß Anspruch 1 behandelt wird.

8. Verfahren nach Anspruch 7, umfassend das In-Kontakt-Bringen der behandelten Stärke mit einer Hefe um Treibstoff oder Trinkalkohol herzustellen.

9. Verfahren nach Anspruch 7, umfassend das Fermentieren der behandelten Stärke in ein Fermentationsprodukt, wie Zitronensäure, Mononatriumglutamat, Gluconsäure, Natriumgluconat, Calciumgluconat, Kaliumgluconat, Gluconodelta-lacton, Natriumerythorbat, Itaconsäure, Milchsäure; Ketone, Aminosäuren, Glutaminsäure (Natriummonoglutamat), Penicillin, Tetracyclin; Enzyme, Vitamine, wie Riboflavin, B12, beta-Carotin oder Hormone.

10. Verfahren zum Herstellen eines Teig-basierten Produktes, umfassend das Hinzufügen des Hybridenzyms gemäß Anspruch 1 zu einem Teig.

## Revendications

1. Enzyme hybride qui comprend une séquence d'acides aminés d'un module catalytique et une séquence d'acides aminés d'un module de liaison de glucides,
a) dans laquelle le module catalytique est une séquence d'alpha-amylase qui consiste en la séquence d'acides aminés présentée dans SEQ ID NO: 8 ; et,
b) dans laquelle le module de liaison de glucides consiste en une séquence d'acides aminés ayant au moins 90% d'identité avec la séquence d'acides aminés de SEQ ID NO: 6.

2. Séquence d'ADN isolé codant pour l'enzyme hybride selon la revendication 1.

3. Construction d'ADN comprenant la séquence d'ADN selon la revendication 2.

4. Vecteur d'expression comprenant la séquence d'ADN selon la revendication 2.

5. Cellule hôte transformée avec un vecteur d'expression selon la revendication 4, ladite cellule hôte étant capable d'exprimer la séquence d'ADN isolé de la revendication 2.

6. Cellule hôte selon la revendication 5, ladite cellule hôte provenant d'un champignon, d'une bactérie, d'une plante ou d'un mammifère.

7. Méthode pour liquéfier l'amidon, dans laquelle un substrat d'amidon gélatinisé ou granulaire est traité dans un milieu aqueux avec l'enzyme hybride selon la revendication 1.

8. Méthode selon la revendication 7 comprenant la mise en contact de l'amidon traité avec une levure pour produire du carburant ou de l'éthanol potable.

9. Méthode selon la revendication 7 comprenant la fermentation de l'amidon traité en un produit de fermentation, tel que l'acide citrique, le glutamate monosodique, l'acide gluconique, le gluconate de sodium, le gluconate de calcium, le gluconate de potassium, le glucono delta-lactone, l'érythorbate de sodium, l'acide itaconique, l'acide lactique, l'acide gluconique; des cétones; des acides aminés, l'acide glutamique (le monoglutaminate de sodium), la pénicilline, la tétracycline; des enzymes ; les vitamines, telles que la riboflavine, B 12, le bêta-carotène ou des hormones.

10. Procédé de préparation d'un produit à base de pâte, comprenant l'ajout de l'enzyme hybride de la revendication 1 à la pâte.
